# EUROPÄISCHE PATENTSCHRIFT

(11) **EP 3 191 586 B1**
(45) Veröffentlichungstag und Bekanntmachung des Hinweises auf die Patenterteilung: **13.11.2019**
(21) Anmeldenummer: 15763296.9
(22) Anmeldetag: 10.09.2015
(51) Int. Cl.: C12N 9/10, C12P 19/02, C12P 19/12, C13K 1/02, C12P 19/18, C13K 11/00

(54) **CELLOBIOSE PHOSPHORYLASE**
CELLOBIOSE PHOSPHORYLASE
CELLOBIOSE PHOSPHORYLASE

(30) Priorität: 10.09.2014 EP 14184301; 10.09.2014 EP 14184302; 29.10.2014 EP 14190891
(43) Veröffentlichungstag der Anmeldung: 19.07.2017
(73) Patentinhaber: Pfeifer & Langen GmbH & Co. KG, 50858 Köln (DE)
(72) Erfinder: KOCH, Timo Johannes, 50189 Elsdorf (DE); HÄSSLER, Thomas, 50968 Köln (DE); BRUCHER, Birgit, 04275 Leipzig (DE); VOGEL, Andreas, 04105 Leipzig (DE)
(74) Vertreter: Kutzenberger Wolff & Partner
(86) Internationale Anmeldenummer: PCT/EP2015/070722
(87) Internationale Veröffentlichungsnummer: WO 2016/038141

(56) Entgegenhaltungen:
- WO-A1-2009/080774
- WO-A1-2012/109274
- US-A- 5 849 529
- MANU R.M. DE GROEVE ET AL: "Construction of cellobiose phosphorylase variants with broadened acceptor specificity towards anomerically substituted glucosides", BIOTECHNOLOGY AND BIOENGINEERING, Bd. 107, Nr. 3, 1. Juni 2010 (2010-06-01), Seiten 413-420, XP055171639, ISSN: 0006-3592, DOI: 10.1002/bit.22818 in der Anmeldung erwähnt
- BERND NIDETZKY ET AL: "Cellobiose phosphorylase from Cellulomonas uda: gene cloning and expression in Escherichia coli, and application of the recombinant enzyme in a 'glycosynthase-type' reaction", JOURNAL OF MOLECULAR CATALYSIS B: ENZYMATIC, Bd. 29, Nr. 1-6, 1. Juni 2004 (2004-06-01) , Seiten 241-248, XP055171347, ISSN: 1381-1177, DOI: 10.1016/j.molcatb.2003.11.014
- TOM DESMET ET AL: "Broadening the synthetic potential of disaccharide phosphorylases through enzyme engineering", PROCESS BIOCHEMISTRY, ELSEVIER, NL, Bd. 47, Nr. 1, 31. Oktober 2011 (2011-10-31), Seiten 11-17, XP028342253, ISSN: 1359-5113, DOI: 10.1016/J.PROCBIO.2011.10.039 [gefunden am 2011-11-10]
- MANU R M DE GROEVE ET AL: "Engineering of cellobiose phosphorylase for glycoside synthesis", JOURNAL OF BIOTECHNOLOGY, ELSEVIER SCIENCE PUBLISHERS, AMSTERDAM, NL, Bd. 156, Nr. 4, 11. Juli 2011 (2011-07-11) , Seiten 253-260, XP028119258, ISSN: 0168-1656, DOI: 10.1016/J.JBIOTEC.2011.07.006 [gefunden am 2011-07-20]
- M.R.M. DE GROEVE ET AL: "Development and application of a screening assay for glycoside phosphorylases", ANALYTICAL BIOCHEMISTRY, Bd. 401, Nr. 1, 1. Juni 2010 (2010-06-01), Seiten 162-167, XP055171387, ISSN: 0003-2697, DOI: 10.1016/j.ab.2010.02.028
- MASAFUMI HIDAKA ET AL: "Structural dissection of the reaction mechanism of cellobiose phosphorylase", BIOCHEMICAL JOURNAL, Bd. 398, Nr. 1, 15. August 2006 (2006-08-15), Seite 37, XP055171470, ISSN: 0264-6021, DOI: 10.1042/BJ20060274

## Beschreibung

Die Erfindung betrifft eine Cellobiose-Phosphorylase, welche u.a. die Synthese von Cellobiose aus Glukose-1-Phosphat und Glukose katalysiert. Die erfindungsgemäße Cellobiose-Phosphorylase kann als Mutante der Cellobiose-Phosphorylase aus *Cellulomonas uda* aufgefasst werden. Im Vergleich zur Cellobiose-Phosphorylase des Wildtyps zeichnet sich die erfindungsgemäße Cellobiose-Phosphorylase durch eine verbesserte Aktivität und Prozessstabilität, insbesondere Temperaturstabilität, sowie eine geringere Edukt- und Produktinhibition aus und ist daher in besonderem Maße zum Einsatz in industriellen Prozessen geeignet.

Cellobiose-Phosphorylasen katalysieren die Spaltung von Cellobiose (D-Glucosyl-β-(1→4)-D-Glukose) in Glukose und Glukose-1-Phosphat. Neben dieser phosphorolytischen Aktivität sind die Enzyme unter geeigneten Bedingungen auch in der Lage, umgekehrt die Synthese von Cellobiose aus Glukose-1-Phosphat und Glukose zu katalysieren.

Cellobiose ist ein natürliches Disaccharid, welches den Grundbaustein für Cellulose bildet. Cellobiose gewinnt zunehmend an Attraktivität für den Lebens- und Futtermittelbereich.

Die Herstellung von Cellobiose kann über chemische oder enzymatische Hydrolyse von Cellulose erfolgen. GB 2 438 573 beschreibt ein Verfahren zur Gewinnung von Cellobiose, welches die Hydrolyse von Cellulose, die Erhöhung des Cellobiose-Anteils in der Zuckerlösung relativ zu weiteren Sacchariden durch Ultrafiltration, sowie anschließende Reinigung der Cellobiose durch Kristallisation umfasst.

Eine Alternative zur Herstellung von Cellobiose aus Cellulose ist die Synthese von Cellobiose unter Verwendung von Cellobiose-Phosphorylasen. Das Schlüsselintermediat dieser Synthese ist Glukose-1-Phosphat, welches entweder durch Phosphorolyse von Stärke mittels einer alpha-Glukan-Phosphorylase oder durch Phosphorolyse von Saccharose mittels einer Saccharose-Phosphorylase erhalten werden kann; letztere spaltet Saccharose in Glukose-1-Phsophat und Fructose.

EP 0 423 768 offenbart ein Verfahren zur Herstellung von Cellobiose ausgehend von Saccharose unter Verwendung einer Saccharose-Phosphorylase, einer Glukose-Isomerase und einer Cellobiose-Phosphorylase. Das Verfahren umfasst folgende Schritte: (1) Spaltung der Saccharose in Anwesenheit von Orthophosphat unter Katalyse von Saccharose-Phosphorylase in Glukose-1-Phosphat und Fructose; (2) Isomerisierung von Fructose in Glukose unter Katalyse von Glukose-Isomerase; (3) Synthese von Cellobiose aus Glukose und Glukose-1-Phosphat unter Katalyse von Cellobiose-Phosphorylase unter Abspaltung von Orthophosphat; (4) teilweise Aufarbeitung der Cellobiose aus dem Reaktionsansatz sowie Rückführung eines Teils des verbleibenden Orthophospat-haltigen Reaktionsansatzes in Schritt (1). Die industrielle Herstellung von Cellobiose ausgehend von Glukose und Glukose-1-Phosphat erfordert eine Cellobiose-Phosphorylase mit hoher Aktivität sowie mit guter Prozess- und Temperaturstabilität.

Eine Möglichkeit zur Optimierung von Enzymen besteht in der Anwendung von Enzym-Engineering, welches auf die Entwicklung von Varianten eines Ausgangsenzyms mit verbesserten Eigenschaften abzielt. Enzym-Engineering wurde bereits auf eine Cellobiose-Phosphorylase aus *Cellulomonas uda* angewandt, mit dem Ziel, die Substratspezifität des Enzyms zu verändern.

De Groeve et al. Protein Engineering, Design & Selection, 2009, 22:393-399 offenbart Enzym-Varianten mit veränderter Substratspezifität, welche eine deutlich erhöhte Aktivität als Lactose-Phosphorylase besitzen und zur Herstellung von Galactose-1-Phosphat eingesetzt werden können.

De Groeve et al., Biotechnology and Bioengineering, 2010, 107:413-420 offenbart weitere Varianten der Cellobiose-Phosphorylase aus *Cellulomonas uda* mit veränderter Substratspezifität, welche eine breitere Akzeptor-Spezifität gegenüber substituierten Glycosiden aufweisen.

Desmet et al., Process Biochemistry, Elsevier, 47, Nr. 1, 2001, 11-17 betrifft die Verbreiterung des synthetischen Potentials von Disaccharid-Phosphorylasen durch Enzym-Engineering.

Nidetzky et al., Journal of Molecular Catalysis B:Enzymatic, 29, Nr. 1-6, 2004, 241-248 betrifft die Cellobiosephosphorylase von Cellulomonas uda, die Expression in E.coli und die Verwendung des rekombinanten Enzyms.

US 5 849 529 betrifft ein Cellobiosephosphorylase-Gen und das entsprechend codierte Protein.

WO 2009/080774 offenbart Lactose-Phosphorylasen, welche durch Mutation der Cellobiose-Phosphorylase aus *Cellulomonas uda* erhalten wurden und zur Herstellung von Galactose-1-Phosphat bzw. von Lactose verwendet werden können.

WO 2012/109274 betrifft Wirtszellen, welche zwei oder mehr rekombinante Cellodextrin- Transporter, eine rekombinante Cellodextrin-Phosphorylase, eine rekombinante ss-Glukosidase, eine rekombinante Phosphoglukomutase, oder eine rekombinante Hexokinase enthalten.

Neben dem Engineering der Cellobiose-Phosphorylase aus *Cellulomonas uda* zur Veränderung der Substratspezifität sind bislang keine weiteren umfangreichen Ansätze zur Optimierung der Cellobiose-Phosphorylase aus *Cellulomonas uda* bzw. anderen Cellobiose-Phosphorylasen durch Engineering bekannt.

Es besteht ein Bedarf an Cellobiose-Phosphorylasen, welche eine effiziente Synthese von Cellobiose aus Glukose-1-Phosphat und Glukose im industriellen Maßstab geeignet sind.

Der Erfindung liegt die Aufgabe zugrunde, eine Cellobiose-Phosphorylase mit verbesserten Eigenschaften bereitzustellen. Im Hinblick auf die Synthese von Cellobiose sollte sich die Cellobiose-Phosphorylase im Vergleich zu bekannten Cellobiose-Phosphorylasen durch eine verbesserte Aktivität und Prozessstabilität, insbesondere Temperaturstabilität, sowie eine geringere Edukt- und Produktinhibition auszeichnen.

Diese Aufgabe wird durch den Gegenstand der Patentansprüche gelöst.

Die Erfindung betrifft eine Cellobiose-Phosphorylase umfassend eine Aminosäuresequenz, welche eine Identität zur Aminosäuresequenz gemäß SEQ ID NO:21 von mindestens 90% aufweist;
wobei die Aminosäure-Sequenz im Vergleich zu SEQ ID NO:1 eine oder mehrere Aminosäure-Mutationen in Positionen 161, 169, 196, 220, 283, 512, 705 aufweist; und
wobei die Aminosäure-Sequenz keine Aminosäure-Mutation K283H umfasst; und wobei die Cellobiose-Phosphorylase im Vergleich zur Cellobiose-Phosphorylase gemäß SEQ ID NO: 1
(i) im Hinblick auf die Umsetzung von Glukose-1-Phosphat mit Glukose zu Cellobiose eine gesteigerte Syntheseaktivität in Gegenwart äquimolarer Mengen Glukose und Glukose-1-Phosphat von 250, 500 und 750 mM; und/oder
(ii) im Hinblick auf die Umsetzung von Glukose-1-Phosphat mit Glukose zu Cellobiose eine höhere Raum-Zeit-Ausbeute pro eingesetzter Enzymmenge in Gegenwart äquimolarer Mengen Glukose und Glukose-1-Phosphat von 250 und 500 mM; und/oder (iii) eine höhere Temperaturstabilität nach Inkubation bei 58 °C für 15 min
aufweist.

In einer bevorzugten Ausführungsform umfasst die erfindungsgemäße Cellobiose-Phosphorylase nicht die Aminosäure A584E und/oder nicht die Aminosäure-Mutation L705Q und/oder nicht die Aminosäure-Mutation L705V und/oder nicht die Aminosäure-Mutation T788E und/oder nicht die Aminosäure-Mutation T788A und/oder nicht die Aminosäure-Mutation T788V; besonders bevorzugt keine der vorstehend genannten Aminosäure-Mutationen.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander
- in einem Sequenzabschnitt D), welcher Positionen 685 bis 745 gemäß SEQ ID NO:1 entspricht; wobei die Aminosäuresequenz der erfindungsgemäßen Cellobiose-Phosphorylase bevorzugt weder die Aminosäure-Mutation L705Q noch die Aminosäure-Mutation L705V aufweist.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander
- in einem Sequenzabschnitt A), welcher Positionen 100 bis 295 gemäß SEQ ID NO:1 entspricht; wobei die Aminosäuresequenz der erfindungsgemäßen Cellobiose-Phosphorylase nicht die Aminosäure-Mutation K283H aufweist; und/oder
- in einem Sequenzabschnitt D), welcher Positionen 685 bis 745 gemäß SEQ ID NO:1 entspricht; wobei die Aminosäuresequenz der erfindungsgemäßen Cellobiose-Phosphorylase bevorzugt weder die Aminosäure-Mutation L705Q noch die Aminosäure-Mutation L705V aufweist.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander
- in einem Sequenzabschnitt D), welcher Positionen 685 bis 745 gemäß SEQ ID NO:1 entspricht; wobei die Aminosäuresequenz der erfindungsgemäßen Cellobiose-Phosphorylase bevorzugt weder die Aminosäure-Mutation L705Q noch die Aminosäure-Mutation L705V aufweist.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander in einem Sequenzabschnitt D), welcher Positionen 685 bis 745 gemäß SEQ ID NO:1 entspricht. Dabei weist die Aminosäuresequenz der erfindungsgemäßen Cellobiose-Phosphorylase bevorzugt weder die Aminosäure-Mutation L705Q noch die Aminosäure-Mutation L705V auf.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander in einem Sequenzabschnitt E), welcher Positionen 755 bis 810 gemäß SEQ ID NO:1 entspricht. Dabei weist die Aminosäuresequenz der erfindungsgemäßen Cellobiose-Phosphorylase bevorzugt weder die Aminosäure-Mutation T788E, noch die Aminosäure-Mutation T788A, noch die Aminosäure-Mutation T788V auf.

Es wurde überraschend gefunden, dass durch Aminosäure-Mutationen in bestimmten Sequenzabschnitten der Aminosäuresequenz der Cellobiose-Phosphorylase aus *Cellulomonas uda* (Wildtyp, SEQ ID NO:1) verbesserte Cellobiose-Phosphorylasen erhältlich sind.

Die erfindungsgemäße Cellobiose-Phosphorylase zeichnet sich im Vergleich zur Cellobiose-Phosphorylase des Wildtyps durch eine verbesserte Aktivität und Prozessstabilität, insbesondere Temperaturstabilität, sowie eine geringere Edukt- und Produktinhibition aus und ist daher in besonderem Maße zum Einsatz in industriellen Prozessen geeignet. Die verbesserten Eigenschaften führen zu einer verbesserten Raum-Zeit-Ausbeute und einem erhöhten Synthese zu Phosphorolyse-Verhältnis.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% aufweist, bevorzugt mindestens 81%, mindestens 82%, mindestens 83%, mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99%.

Erfindungsgemäß ist "Identität" definiert als der Prozentsatz identischer Übereinstimmungen zwischen zwei Vergleichssequenzen bei optimaler Ausrichtung (*alignment*). Zur optimalen Ausrichtung können Lücken in jeder der beiden Sequenzen eingeführt werden. Bevorzugt wird die Identität zwischen zwei Vergleichssequenzen unter Verwendung des Smith and Waterman-Algorithmus bestimmt (Smith TF, Waterman MS J (1981) Mol. Biol. 147, 195-197), bevorzugt unter Verwendung des Computerprogramms WATER aus dem EMBOSS-Paket, welches frei verfügbar ist und den Smith und Waterman-Algorithmus implementiert hat (Reis P, Longden I, Bleasby A (2000) Trends in Genetics 16, 276-277). Dabei wird bevorzugt BLOSUM62 für die Substitutionsmatrix mit einem GOP (*gap opening penalty*) von 10 und einem GEP (*gap extension penalty*) von 0,5 verwendet.

Eine Aminosäure-Mutation im Sinne dieser Erfindung ist definiert als Austausch der Aminosäure einer Cellobiose-Phosphorylase Wildtypsequenz, bevorzugt der Cellobiose-Phosphorylase Wildtypsequenz gemäß SEQ ID NO:1 in eine andere proteinogene Aminosäure.

Die vorliegende Anmeldung beschreibt eine Cellobiose-Phosphorylase mit einem Sequenzabschnitt A) Positionen 155 bis 289 gemäß SEQ ID NO:1. Bevorzugt ist Sequenzabschnitt A) aufgeteilt in Sequenzabschnitte A₁), A₂), A₃), A₄), A₅), A₆), A₇), A₈) und A₉), wobei die erfindungsgemäße Cellobiose-Phosphorylase, wenn sie im Vergleich zu SEQ ID NO:1 wenigstens eine Aminosäure-Mutation, bevorzugt wenigstens zwei oder wenigstens drei Aminosäure-Mutationen in Sequenzabschnitt A) umfasst, diese wenigstens eine Aminosäure-Mutation, bevorzugt wenigstens zwei oder wenigstens drei Aminosäure-Mutationen jeweils unabhängig voneinander bevorzugt in einem der Sequenzabschnitte A₁), A₂), A₃), A₄), A₅), A₆), A₇), A₈) und A₉) umfasst, und wobei
- Sequenzabschnitt A₁) Positionen 155 bis 167 gemäß SEQ ID NO:1 entspricht;
- Sequenzabschnitt A₂) Positionen 158 bis 170 gemäß SEQ ID NO:1 entspricht;
- Sequenzabschnitt A₃) Positionen 160 bis 172 gemäß SEQ ID NO:1 entspricht;
- Sequenzabschnitt A₄) Positionen 163 bis 175 gemäß SEQ ID NO:1 entspricht;
- Sequenzabschnitt A₅) Positionen 164 bis 176 gemäß SEQ ID NO:1 entspricht;
- Sequenzabschnitt A₆) Positionen 182 bis 194 gemäß SEQ ID NO:1 entspricht;
- Sequenzabschnitt A₇) Positionen 190 bis 202 gemäß SEQ ID NO:1 entspricht;
- Sequenzabschnitt A₈) Positionen 214 bis 226 gemäß SEQ ID NO:1 entspricht; und
- Sequenzabschnitt A₉) Positionen 277 bis 289 gemäß SEQ ID NO:1 entspricht.

Bevorzugt umfasst die Cellobiose-Phosphorylase im Vergleich zu SEQ ID NO:1 wenigstens eine Aminosäure-Mutation, bevorzugt wenigstens zwei oder wenigstens drei Aminosäure-Mutationen, jeweils unabhängig voneinander in Sequenzabschnitt A), wobei diese wenigstens eine Aminosäure-Mutation, bevorzugt wenigstens zwei oder wenigstens drei Aminosäure-Mutationen, jeweils unabhängig voneinander bevorzugt in einem der Sequenzabschnitte A₁), A₂), A₃), A₄), A₅), A₇), A₈) und A₉) liegt bzw. liegen, bevorzugt nicht jedoch in Sequenzabschnitt A₆).

In einer beschrieben Form umfasst die Cellobiose-Phosphorylase im Vergleich zu SEQ ID NO:1 keine Aminosäure-Mutation in Position 188 und/oder keine Aminosäure-Mutation in Position 283 und/oder keine Aminosäure-Mutation in Position 584 und/oder keine Aminosäure-Mutation in Position 788.

In bevorzugten Ausführungsformen weist die Aminosäuresequenz der erfindungsgemäßen Cellobiose-Phosphorylase im Vergleich zu SEQ ID NO:1
(i) bevorzugt keine Aminosäure-Mutation in Position 188 und/oder keine Aminosäure-Mutation in Position 283 und/oder keine Aminosäure-Mutation in Position 584 und/oder keine Aminosäure-Mutation in Position 705 auf; besonders bevorzugt keine der vorstehend genannten Aminosäure-Mutationen.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander
- in einem Sequenzabschnitt A₁), welcher Positionen 155 bis 167 gemäß SEQ ID NO:1 entspricht, bevorzugt Positionen 160 bis 162; und/oder
- in einem Sequenzabschnitt A₂), welcher Positionen 158 bis 170 gemäß SEQ ID NO:1 entspricht, bevorzugt Positionen 163 bis 164; und/oder
- in einem Sequenzabschnitt A₃), welcher Positionen 160 bis 172 gemäß SEQ ID NO:1 entspricht, bevorzugt Positionen 165 bis 167; und/oder
- in einem Sequenzabschnitt A₄), welcher Positionen 163 bis 175 gemäß SEQ ID NO:1 entspricht, bevorzugt Positionen 168 bis 169; und/oder
- in einem Sequenzabschnitt A₅), welcher Positionen 164 bis 176 gemäß SEQ ID NO:1 entspricht, bevorzugt Positionen 170 bis 171; und/oder
- in einem Sequenzabschnitt A₇), welcher Positionen 190 bis 202 gemäß SEQ ID NO:1 entspricht; und/oder
- in einem Sequenzabschnitt A₈), welcher Positionen 214 bis 226 gemäß SEQ ID NO:1 entspricht; und/oder
- in einem Sequenzabschnitt A₉), welcher Positionen 277 bis 289 gemäß SEQ ID NO:1 entspricht; wobei die Aminosäuresequenz der erfindungsgemäßen Cellobiose-Phosphorylase nicht die Aminosäure-Mutation K283H aufweist; und/oder
- in einem Sequenzabschnitt D), welcher Positionen 685 bis 745 gemäß SEQ ID NO:1 entspricht; wobei die Aminosäuresequenz der erfindungsgemäßen Cellobiose-Phosphorylase bevorzugt weder die Aminosäure-Mutation L705Q noch die Aminosäure-Mutation L705V aufweist.

In einer einer beschriebenen Form umfasst die Cellobiose-Phosphorylase dabei ggf. zusätzlich eine weitere Aminosäure-Mutation in einem Sequenzabschnitt A₆), welcher Positionen 182 bis 194 gemäß SEQ ID NO:1 entspricht, bevorzugt Positionen 187 bis 189. In einer anderen beschriebenen Form umfasst die Cellobiose-Phosphorylase dabei ggf. zusätzlich keine weitere Aminosäure-Mutation in einem Sequenzabschnitt A₆), welcher Positionen 182 bis 194 gemäß SEQ ID NO:1 entspricht, bevorzugt Positionen 187 bis 189.

Die vorliegende Anmeldung beschreibt eine Cellobiose-Phosphorylase mit einer Aminosäuresequenz, welche eine Identität zur Aminosäuresequenz gemäß SEQ ID NO:1 von mindestens 80% oder mindestens 81%, bevorzugter mindestens 82% oder mindestens 83% und insbesondere mindestens 84%, mindestens 85%, mindestens 86%, mindestens 87%, mindestens 88%, mindestens 89%, mindestens 90%, mindestens 91%, mindestens 92%, mindestens 93%, mindestens 94%, mindestens 95%, mindestens 96%, mindestens 97%, mindestens 98% oder mindestens 99% aufweist und welche im Vergleich zu SEQ ID NO:1 wenigstens eine Aminosäure-Mutation, bevorzugt wenigstens zwei oder wenigstens drei Aminosäure-Mutationen umfasst jeweils unabhängig voneinander
- in einem Sequenzabschnitt A₁), welcher Positionen 155 bis 167 gemäß SEQ ID NO:1 entspricht, bevorzugt Positionen 160 bis 162; und/oder
- in einem Sequenzabschnitt A₂), welcher Positionen 158 bis 170 gemäß SEQ ID NO:1 entspricht, bevorzugt Positionen 163 bis 164; und/oder
- in einem Sequenzabschnitt A₃), welcher Positionen 160 bis 172 gemäß SEQ ID NO:1 entspricht, bevorzugt Positionen 165 bis 167; und/oder
- in einem Sequenzabschnitt A₄), welcher Positionen 163 bis 175 gemäß SEQ ID NO:1 entspricht, bevorzugt Positionen 168 bis 169; und/oder
- in einem Sequenzabschnitt A₅), welcher Positionen 164 bis 176 gemäß SEQ ID NO:1 entspricht, bevorzugt Positionen 170 bis 171; und/oder
- in einem Sequenzabschnitt A₇), welcher Positionen 190 bis 202 gemäß SEQ ID NO:1 entspricht; und/oder
- in einem Sequenzabschnitt A₈), welcher Positionen 214 bis 226 gemäß SEQ ID NO:1 entspricht; und/oder
- in einem Sequenzabschnitt A₉), welcher Positionen 277 bis 289 gemäß SEQ ID NO:1 entspricht; wobei die Aminosäuresequenz der erfindungsgemäßen Cellobiose-Phosphorylase nicht die Aminosäure-Mutation K283H aufweist; und/oder
- in einem Sequenzabschnitt D), welcher Positionen 685 bis 745 gemäß SEQ ID NO:1 entspricht; wobei die Aminosäuresequenz der erfindungsgemäßen Cellobiose-Phosphorylase bevorzugt weder die Aminosäure-Mutation L705Q noch die Aminosäure-Mutation L705V aufweist.

In einer beschriebenen Form umfasst die Cellobiose-Phosphorylase dabei ggf. zusätzlich eine weitere Aminosäure-Mutation in einem Sequenzabschnitt A₆), welcher Positionen 182 bis 194 gemäß SEQ ID NO:1 entspricht, bevorzugt Positionen 187 bis 189. Die Cellobiose-Phosphorylase umfasst dabei ggf. zusätzlich keine weitere Aminosäure-Mutation in einem Sequenzabschnitt A₆), welcher Positionen 182 bis 194 gemäß SEQ ID NO:1 entspricht, bevorzugt Positionen 187 bis 189. Die Cellobiose-Phosphorylase umfasst dabei ggf. zusätzlich eine weitere Aminosäure-Mutation in einem Sequenzabschnitt E), welcher Positionen 755 bis 810 gemäß SEQ ID NO:1 entspricht. Die Cellobiose-Phosphorylase umfasst dabei ggf. zusätzlich keine weitere Aminosäure-Mutation in einem Sequenzabschnitt E), welcher Positionen 755 bis 810 gemäß SEQ ID NO:1 entspricht.

Die beschriebene Cellobiose-Phosphorylase entspricht
- Sequenzabschnitt A₁) Positionen 155 bis 167, bevorzugt Positionen 156 bis 166, bevorzugter Positionen 157 bis 165, noch bevorzugter Positionen 158 bis 164, am bevorzugtesten Positionen 159 bis 163, und insbesondere Positionen 160 bis 162, gemäß SEQ ID NO:1; und/oder
- Sequenzabschnitt A₂) Positionen 158 bis 170, bevorzugt Positionen 159 bis 169, bevorzugter Positionen 160 bis 168, noch bevorzugter Positionen 161 bis 167, am bevorzugtesten Positionen 162 bis 166, und insbesondere Positionen 163 bis 165, gemäß SEQ ID NO:1; und/oder
- Sequenzabschnitt A₃) Positionen 160 bis 172, bevorzugt Positionen 161 bis 171, bevorzugter Positionen 162 bis 170, noch bevorzugter Positionen 163 bis 169, am bevorzugtesten Positionen 164 bis 168, und insbesondere Positionen 165 bis 167, gemäß SEQ ID NO:1; und/oder
- Sequenzabschnitt A₄) Positionen 163 bis 175, bevorzugt Positionen 164 bis 174, bevorzugter Positionen 165 bis 173, noch bevorzugter Positionen 166 bis 172, am bevorzugtesten Positionen 167 bis 171, und insbesondere Positionen 168 bis 170, gemäß SEQ ID NO:1; und/oder
- Sequenzabschnitt A₅) Positionen 164 bis 176, bevorzugt Positionen 165 bis 175, bevorzugter Positionen 166 bis 174, noch bevorzugter Positionen 167 bis 173, am bevorzugtesten Positionen 168 bis 172, und insbesondere Positionen 169 bis 171, gemäß SEQ ID NO:1; und/oder
- Sequenzabschnitt A₆) Positionen 182 bis 194, bevorzugt Positionen 183 bis 193, bevorzugter Positionen 184 bis 192, noch bevorzugter Positionen 185 bis 191, am bevorzugtesten Positionen 186 bis 190, und insbesondere Positionen 187 bis 189, gemäß SEQ ID NO:1; und/oder
- Sequenzabschnitt A₇) Positionen 190 bis 202, bevorzugt Positionen 191 bis 201, bevorzugter Positionen 192 bis 200, noch bevorzugter Positionen 193 bis 199, am bevorzugtesten Positionen 194 bis 198, und insbesondere Positionen 195 bis 197, gemäß SEQ ID NO:1; und/oder
- Sequenzabschnitt A₈) Positionen 214 bis 226, bevorzugt Positionen 215 bis 225, bevorzugter Positionen 216 bis 224, noch bevorzugter Positionen 217 bis 223, am bevorzugtesten Positionen 218 bis 222, und insbesondere Positionen 219 bis 221, gemäß SEQ ID NO:1; und/oder
- Sequenzabschnitt A₉) Positionen 277 bis 289, bevorzugt Positionen 278 bis 288, bevorzugter Positionen 279 bis 287, noch bevorzugter Positionen 280 bis 286, am bevorzugtesten Positionen 281 bis 285, und insbesondere Positionen 282 bis 284, gemäß SEQ ID NO:1; und/oder
- Sequenzabschnitt B) Positionen 350 bis 362, bevorzugt Positionen 351 bis 361, bevorzugter Positionen 352 bis 360, noch bevorzugter Positionen 353 bis 359, am bevorzugtesten Positionen 354 bis 358, und insbesondere Positionen 355 bis 357, gemäß SEQ ID NO:1; und/oder
- Sequenzabschnitt C) Positionen 578 bis 590, bevorzugt Positionen 579 bis 589, bevorzugter Positionen 580 bis 588, noch bevorzugter Positionen 581 bis 587, am bevorzugtesten Positionen 582 bis 586, und insbesondere Positionen 583 bis 585, gemäß SEQ ID NO:1; und/oder
- Sequenzabschnitt D) Positionen 699 bis 711, bevorzugt Positionen 700 bis 710, bevorzugter Positionen 701 bis 709, noch bevorzugter Positionen 702 bis 708, am bevorzugtesten Positionen 703 bis 707, und insbesondere Positionen 704 bis 706, gemäß SEQ ID NO:1; und/oder
- Sequenzabschnitt E) Positionen 782 bis 794, bevorzugt Positionen 783 bis 793, bevorzugter Positionen 784 bis 792, noch bevorzugter Positionen 785 bis 791, am bevorzugtesten Positionen 786 bis 790, und insbesondere Positionen 787 bis 789, gemäß SEQ ID NO:1.

Die beschriebene Cellobiose-Phosphorylase umfasst, wenn sie im Vergleich zu SEQ ID NO:1 eine Aminosäure-Mutation in Sequenzabschnitt A₆) umfasst, zusätzlich wenigstens eine weitere Aminosäure-Mutation, bevorzugt allerdings nicht in Sequenzabschnitt A₉).

Bevorzugt ist wenigstens eine Aminosäure-Mutation ausgewählt aus der Gruppe bestehend aus
- Q161, S169, D196, A220, K283; und
- L705.

Besonders bevorzugt ist wenigstens eine Aminosäure-Mutation ausgewählt aus der Gruppe bestehend aus
- Q161, S169, A220, K283, und
- L705.

Dabei sind die bevorzugten Austausche für diese wenigstens eine Aminosäure-Mutation in diesen Positionen: Q161M, Q161A, Q161G, Q161I, Q161L, oder Q161V; S169V, S169A, S169G, S169I, S169L, oder S169M; A220L, A220G, A220I, A220L, A220M, oder A220V; K283A, K283G, K283I, K283L, K283M, oder K283V; L705T, L705N, L705C, L705Q, oder L705S; besonders bevorzugt Q161M, S169V, A220L, K283A, bzw. L705T.

Besonders bevorzugt ist ggf. zusätzlich zu der vorstehend genannten, wenigstens einen Aminosäure-Mutation wenigstens eine weitere Aminosäure-Mutation ausgewählt aus der Gruppe bestehend aus
- Y164, R166, I170, R188, D196, und
- F356 und
- ggf. A512.

Dabei sind die bevorzugten Austausche für diese wenigstens eine weitere Aminosäure-Mutation in diesen Positionen: Y164F, oder Y164W; R166K, R166H, R166M, R166A, R166G, R166I, R166L, oder R166V; I170T, I170N, I170C, I170Q, oder I170S; R188K, oder R188H; D196N, D196C, D196Q, D196S, oder D196T; besonders bevorzugt Y164F, R166M, I170T, R188K, D196N bzw. F356I (und ggf. A512V).

Bevorzugt umfasst die Cellobiose-Phosphorylase im Vergleich zu SEQ ID NO:1 wenigstens zwei, drei, vier, fünf, sechs, sieben, acht oder neun Aminosäure-Mutationen, welche jeweils unabhängig voneinander wie vorstehend definiert sind.

In einer beschriebenen Form liegen die wenigstens zwei, drei, vier, fünf, sechs, sieben, acht oder neun Aminosäure-Mutationen unabhängig voneinander in Sequenzabschnitt A₁), A₂), A₃), A₄), A₅), A₇), A₈), A₉) und/oder D). In einer beschriebenen Form liegt keine Aminosäure-Mutationen in Sequenzabschnitt A₆).

In einer beschriebenen Form liegen die wenigstens zwei, drei, vier, oder fünf Aminosäure-Mutationen unabhängig voneinander in Sequenzabschnitten A₁), A₇), A₈) und/oder D), wobei bevorzugt eine Aminosäure-Mutation in Sequenzabschnitt A₁), eine Aminosäure-Mutation in Sequenzabschnitt A₇), eine Aminosäure-Mutation in Sequenzabschnitt A₈), und/oder eine Aminosäure-Mutation in Sequenzabschnitt D) liegt.

In einer beschriebenen Form liegen wenigstens zwei, drei, vier oder fünf Aminosäure-Mutationen unabhängig voneinander in Sequenzabschnitt A₁), A₈), A₉), und/oder D), wobei bevorzugt eine Aminosäure-Mutation in Sequenzabschnitt A₁), eine Aminosäure-Mutation in Sequenzabschnitt A₈), eine Aminosäure-Mutation in Sequenzabschnitt A₉), und/oder eine Aminosäure-Mutation in Sequenzabschnitt D) liegt. In einer beschriebenen Form liegt allerdings keine Aminosäure-Mutationen in Sequenzabschnitt A₆). In einer anderen beschriebenen Form liegen darüber hinaus eine oder mehrere weitere Aminosäure-Mutationen unabhängig voneinander in Sequenzabschnitten A₇), und/oder F), wobei bevorzugt eine Aminosäure-Mutation in Sequenzabschnitt A₇), und/oder eine Aminosäure-Mutation in Sequenzabschnitt F) liegt.

In einer beschriebenen Form liegen die wenigstens zwei, drei, vier, fünf, sechs, sieben, acht oder neun Aminosäure-Mutationen unabhängig voneinander in Sequenzabschnitt D).

In einer beschriebenen Form liegen die wenigstens zwei, drei, vier, fünf, sechs, sieben, acht oder neun Aminosäure-Mutationen in Sequenzabschnitt A), bevorzugter unabhängig voneinander in Sequenzabschnitten A₁), A₂), A₃), A₄), A₅), A₇), A₈) und/oder A₉). In einer beschriebenen Form liegt keine Aminosäure-Mutation in Sequenzabschnitt A₆).

In beschriebenen Formen umfasst die erfindungsgemäße Cellobiose-Phosphorylase im Vergleich zu SEQ ID NO:1 wenigstens zwei Aminosäure-Mutationen *"erste Aminosäure-Mutation in Sequenzabschnitt"*//*"zweite Aminosäure-Mutation in Sequenzabschnitt"* ausgewählt aus der Gruppe bestehend aus: A₁//A₂, Aᵢ//A₃, A₁//A₄, A₁//A₅, A₁//A₇, A₁//A₈, A₁//A₉, A₁/D;A₂//A₂, A₂//A₃, A₂//A₄, A₂//A₅, A₂//A₇, A₂//A₈, A₂//A₉A₂//D; A₃//A₃, A₃//A₄, A₃//A₅, A₃//A₇, A₃//A₈, A₃//A₉, A₃//D; A₄//A₅, A₄//A₇, A₄//A₈, A₄//A₉, A₄//D; A₅//A₇, A₅//A₈, A₅//A₉, A₅//D;; A₇//A₈, A₇//A₉, A₇//D; A₈//A₉,, A₈//D; A₉//D. Dabei bedeutet beispielsweise "A₈//A₉", dass die erste der wenigstens zwei Aminosäure-Mutationen im Sequenzabschnitt A₈) liegt und die zweite der wenigstens zwei Aminosäure-Mutationen im Sequenzabschnitt A₉) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in einem der Sequenzabschnitte liegt ausgewählt aus der Gruppe der Sequenzabschnitte A₂), A₃), A₄), A₈) und D).

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt A₁) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt A₂) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt A₃) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt A₄) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt A₈) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt B) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt D) liegt; wobei die Aminosäuresequenz der erfindungsgemäßen Cellobiose-Phosphorylase bevorzugt weder die Aminosäure-Mutation L705Q noch die Aminosäure-Mutation L705V aufweist.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₁) und die andere Aminosäure-Mutation in Sequenzabschnitt E) liegt; wobei die Aminosäuresequenz der erfindungsgemäßen Cellobiose-Phosphorylase bevorzugt weder die Aminosäure-Mutation T788E, noch die Aminosäure-Mutation T788A, noch die Aminosäure-Mutation T788V aufweist.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in einem der Sequenzabschnitte liegt ausgewählt aus der Gruppe der Sequenzabschnitte A₁), A₂), A₃), A₄) und D).

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt A₁) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt A₂) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt A₃) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt A₄) liegt.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt B) liegt.

beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt D) liegt; wobei die Aminosäuresequenz der erfindungsgemäßen Cellobiose-Phosphorylase bevorzugt weder die Aminosäure-Mutation L705Q noch die Aminosäure-Mutation L705V aufweist.

In beschriebenen Formen umfasst die Cellobiose-Phosphorylase im Vergleich zu SEQ ID NO:1 wenigstens zwei Aminosäure-Mutationen, von denen eine Aminosäure-Mutation in Sequenzabschnitt A₈) und die andere Aminosäure-Mutation in Sequenzabschnitt E) liegt; wobei die Aminosäuresequenz der erfindungsgemäßen Cellobiose-Phosphorylase bevorzugt weder die Aminosäure-Mutation T788E, noch die Aminosäure-Mutation T788A, noch die Aminosäure-Mutation T788V aufweist.

In beschriebenen Formen umfasst die Cellobiose-Phosphorylase im Vergleich zu SEQ ID NO:1 wenigstens drei Aminosäure-Mutationen, von denen eine Aminosäure-Mutation in Sequenzabschnitt A₁), eine Aminosäure-Mutation in Sequenzabschnitt A₄) und eine Aminosäure-Mutation in Sequenzabschnitt A₈) liegt.

In beschriebenen Formen umfasst die Cellobiose-Phosphorylase im Vergleich zu SEQ ID NO:1 wenigstens drei Aminosäure-Mutationen, von denen eine Aminosäure-Mutation in Sequenzabschnitt A₁), eine Aminosäure-Mutation in Sequenzabschnitt A₈) und eine Aminosäure-Mutation in Sequenzabschnitt D) liegt, wobei die Aminosäuresequenz der erfindungsgemäßen Cellobiose-Phosphorylase bevorzugt weder die Aminosäure-Mutation L705Q noch die Aminosäure-Mutation L705V aufweist.

In beschriebenen Formen umfasst die Cellobiose-Phosphorylase im Vergleich zu SEQ ID NO:1 wenigstens vier Aminosäure-Mutationen, von denen eine Aminosäure-Mutation in Sequenzabschnitt A₁), eine Aminosäure-Mutation in Sequenzabschnitt A₄) eine Aminosäure-Mutation in Sequenzabschnitt A₈) und eine Aminosäure-Mutation in Sequenzabschnitt D) liegt, wobei die Aminosäuresequenz der Cellobiose-Phosphorylase bevorzugt weder die Aminosäure-Mutation L705Q noch die Aminosäure-Mutation L705V aufweist.

In beschriebenen Formen umfasst die Cellobiose-Phosphorylase im Vergleich zu SEQ ID NO:1 wenigstens vier Aminosäure-Mutationen, von denen eine Aminosäure-Mutation in Sequenzabschnitt A₁), eine Aminosäure-Mutation in Sequenzabschnitt A₈), eine Aminosäure-Mutation in Sequenzabschnitt A₉) und eine Aminosäure-Mutation in Sequenzabschnitt D) liegt wobei die Aminosäuresequenz der Cellobiose-Phosphorylase nicht die Aminosäure-Mutation K283H und bevorzugt weder die Aminosäure-Mutation L705Q noch die Aminosäure-Mutation L705V aufweist.

In beschriebenen Formen umfasst die Cellobiose-Phosphorylase im Vergleich zu SEQ ID NO:1 wenigstens fünf Aminosäure-Mutationen, von denen eine Aminosäure-Mutation in Sequenzabschnitt A₁), eine Aminosäure-Mutation in Sequenzabschnitt A₄), eine Aminosäure-Mutation in Sequenzabschnitt A₈), eine Aminosäure-Mutation in Sequenzabschnitt A₉) und eine Aminosäure-Mutation in Sequenzabschnitt D) liegt, wobei die Aminosäuresequenz der Cellobiose-Phosphorylase nicht die Aminosäure-Mutation K283H und bevorzugt weder die Aminosäure-Mutation L705Q noch die Aminosäure-Mutation L705V aufweist.

In beschriebenen Formen umfasst die Cellobiose-Phosphorylase im Vergleich zu SEQ ID NO:1 wenigstens fünf Aminosäure-Mutationen, von denen eine Aminosäure-Mutation in Sequenzabschnitt A₁), eine Aminosäure-Mutation in Sequenzabschnitt A₈), eine Aminosäure-Mutation in Sequenzabschnitt A₉), eine Aminosäure-Mutation in Sequenzabschnitt D) und eine Aminosäure-Mutation in Sequenzabschnitt E) liegt, wobei die Aminosäuresequenz der Cellobiose-Phosphorylase bevorzugt weder die Aminosäure-Mutation K283H noch die Aminosäure-Mutation L705Q noch die Aminosäure-Mutation L705V noch die Aminosäure-Mutation T788E noch die Aminosäure-Mutation T788A aufweist.

In beschriebenen Formen umfasst die Cellobiose-Phosphorylase im Vergleich zu SEQ ID NO:1 wenigstens sechs Aminosäure-Mutationen, von denen eine Aminosäure-Mutation in Sequenzabschnitt A₁), eine Aminosäure-Mutation in Sequenzabschnitt A₄), eine Aminosäure-Mutation in Sequenzabschnitt A₈), eine Aminosäure-Mutation in Sequenzabschnitt A₉), eine Aminosäure-Mutation in Sequenzabschnitt D) und eine Aminosäure-Mutation in Sequenzabschnitt E) liegt, wobei die Aminosäuresequenz der Cellobiose-Phosphorylase bevorzugt weder die Aminosäure-Mutation K283H noch die Aminosäure-Mutation L705Q noch die Aminosäure-Mutation L705V noch die Aminosäure-Mutation T788E noch die Aminosäure-Mutation T788A aufweist.

In einer bevorzugten Ausführungsform ist die wenigstens eine Aminosäure-Mutation ausgewählt aus der Gruppe bestehend aus
- Q161M, Q161A, Q161G, Q161I, Q161L, Q161V, S169V, S169A, S169G, S169I, S169L, S169M, D196N, D196C, D196Q, D196S, D196T, A220L, A220G, A220I, A220L, A220M, A220V, K283A, K283G, K283I, K283L, K283M, K283V;
- L705T, L705N, L705C, L705Q, und L705S.

In einer bevorzugten Ausführungsform ist die wenigstens eine Aminosäure-Mutation ausgewählt aus der Gruppe bestehend aus
- Q161M, S169V, D196N, A220L, K283A; und
- L705T.

In einer beschriebenen Form umfasst die Cellobiose-Phosphorylase im Vergleich zu SEQ ID NO:1 wenigstens eine weitere Aminosäure-Mutation im Sequenzabschnitt F), welcher Positionen 375 bis 555 gemäß SEQ ID NO:1 entspricht. Bevorzugt ist diese wenigstens eine weitere Aminosäure-Mutation an Position 512 (A512).

Die Cellobiose-Phosphorylase umfasst bevorzugt im Vergleich zu SEQ ID NO:1 nicht gleichzeitig die Aminosäure-Mutation R188K und die Aminosäure-Mutation K283H.

Die Cellobiose-Phosphorylase umfasst bevorzugt im Vergleich zu SEQ ID NO:1 nicht gleichzeitig die Aminosäure-Mutation T788V und die Aminosäure-Mutation K283H.

In einer beschriebenen Form umfasst die Cellobiose-Phosphorylase im Vergleich zu SEQ ID NO:1 keine Aminosäure-Mutation R188T.

In einer erfindungsgemäßen Ausführungsform umfasst die Cellobiose-Phosphorylase im Vergleich zu SEQ ID NO:1 keine Aminosäure-Mutation K283H.

In einer beschriebenen Form umfasst die Cellobiose-Phosphorylase im Vergleich zu SEQ ID NO:1 keine Aminosäure-Mutation A512T und/oder keine Aminosäure-Mutation A512P und/oder keine Aminosäure-Mutation A512S.

In einer beschriebenen Form umfasst die Cellobiose-Phosphorylase im Vergleich zu SEQ ID NO:1 keine Aminosäure-Mutation A584E.

In einer beschriebenen Form umfasst die Cellobiose-Phosphorylase im Vergleich zu SEQ ID NO:1 keine Aminosäure-Mutation L705Q und/oder keine Aminosäure-Mutation L705V.

In einer beschriebenen Form umfasst die Cellobiose-Phosphorylase im Vergleich zu SEQ ID NO:1 keine Aminosäure-Mutation T788E und/oder keine Aminosäure-Mutation T788A.

Beschrieben sind Cellobiose-Phosphorylasen mit einer Aminosäure-Sequenz ausgewählt aus der Gruppe bestehend aus SEQ ID NO: 2, 3, 4, 5, 6, 7, 8, 9, 10, 11, 12, 13, 14, 15, 16, 17, 18, 19, 20, 21, 22, 23, 24, 25, 26, 27 und 28.

In einer beschriebenen Form umfasst die Cellobiose-Phosphorylase eine Aminosäure-Sequenz, welche sich von SEQ ID NO:1 unterscheidet und welche eine Identität zur Aminosäuresequenz gemäß SEQ ID NO:8 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: 161, 188, 196, 220 und 705. Bevorzugt weist diese Aminosäure-Sequenz im Vergleich zu SEQ ID NO:1 eine oder mehrere der folgenden Aminosäure-Mutationen auf, vorzugsweise alle davon: Q161M, R188K, D196N, A220L und L705T.

In einer beschriebenen Form umfasst die Cellobiose-Phosphorylase eine Aminosäure-Sequenz, welche sich von SEQ ID NO:1 unterscheidet und welche eine Identität zur Aminosäuresequenz gemäß SEQ ID NO:18 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: 161, 164, 188, 196, 220, 283, 512, und 705. Bevorzugt weist diese Aminosäure-Sequenz im Vergleich zu SEQ ID NO:1 eine oder mehrere der folgenden Aminosäure-Mutationen auf, vorzugsweise alle davon: Q161M, Y164F, R188K, D196N, A220L, K283A, A512V und L705T.

In einer beschriebenen Form umfasst die Cellobiose-Phosphorylase eine Aminosäure-Sequenz, welche sich von SEQ ID NO:1 unterscheidet und welche eine Identität zur Aminosäuresequenz gemäß SEQ ID NO:19 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: 161, 164, 188, 196, 220, 283, 512, 705 und 788. Bevorzugt weist diese Aminosäure-Sequenz im Vergleich zu SEQ ID NO:1 eine oder mehrere der folgenden Aminosäure-Mutationen auf, vorzugsweise alle davon: Q161M, Y164F, R188K, D196N, A220L, K283A, A512V, L705T und T788V.

In einer beschriebenen Form umfasst die Cellobiose-Phosphorylase eine Aminosäure-Sequenz, welche sich von SEQ ID NO:1 unterscheidet und welche eine Identität zur Aminosäuresequenz gemäß SEQ ID NO:20 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist diese Aminosäure-Sequenz im Vergleich zu SEQ ID NO:1 eine oder mehrere Aminosäure-Mutationen in folgenden Positionen auf, vorzugsweise alle davon: 161, 166, 188, 196, 220, 283, 512, 705 und 788. Bevorzugt weist diese Aminosäure-Sequenz im Vergleich zu SEQ ID NO:1 eine oder mehrere der folgenden Aminosäure-Mutationen auf, vorzugsweise alle davon: Q161M, R166M, R188K, D196N, A220L, K283A, A512V, L705T und T788V.

Erfindungsgemäß umfasst die Cellobiose-Phosphorylase eine Aminosäure-Sequenz, welche sich von SEQ ID NO:1 unterscheidet und welche eine Identität zur Aminosäuresequenz gemäß SEQ ID NO:21 von mindestens 90%, mindestens 91%, mindestens 92%, mindestens 93%, mindestens 94%, mindestens 95%, mindestens 96%, mindestens 97%, mindestens 98% oder mindestens 99% aufweist. Erfindungsgemäß weist diese Aminosäure-Sequenz im Vergleich zu SEQ ID NO:1 eine oder mehrere Aminosäure-Mutationen in folgenden Positionen auf, vorzugsweise alle davon: 161, 169, 196, 220, 283, 512 und 705. Bevorzugt weist diese Aminosäure-Sequenz im Vergleich zu SEQ ID NO:1 eine oder mehrere der folgenden Aminosäure-Mutationen auf, vorzugsweise alle davon: Q161M, S169V, D196N, A220L, K283A, A512V und L705T.

In einer beschriebenen Form umfasst die Cellobiose-Phosphorylase eine Aminosäure-Sequenz, welche sich von SEQ ID NO:1 unterscheidet und welche eine Identität zur Aminosäuresequenz gemäß SEQ ID NO:25 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist die Aminosäure-Sequenz im Vergleich zu SEQ ID NO:1 eine oder mehrere Aminosäure-Mutationen in Positionen 161, 169, 188, 196, 220, 283, 633, 705 und 788 auf. Bevorzugt weist die Aminosäure-Sequenz im Vergleich zu SEQ ID NO:1 eine oder mehrere der Aminosäure-Mutationen Q161M, S169V, R188K, D196N, A220L, K283A, M633I, L705T und L788V auf.

In einer beschriebenen Form umfasst die Cellobiose-Phosphorylase eine Aminosäure-Sequenz, welche sich von SEQ ID NO:1 unterscheidet und welche eine Identität zur Aminosäuresequenz gemäß SEQ ID NO:26 von mindestens 90%, mindestens 91%, mindestens 92%, mindestens 93%, mindestens 94%, mindestens 95%, mindestens 96%, mindestens 97%, mindestens 98% oder mindestens 99% aufweist. Bevorzugt weist die Aminosäure-Sequenz im Vergleich zu SEQ ID NO:1 eine oder mehrere Aminosäure-Mutationen in Positionen 161, 169, 196, 220, 283, 633, 705 und 788 auf. Bevorzugt weist die Aminosäure-Sequenz im Vergleich zu SEQ ID NO:1 eine oder mehrere der Aminosäure-Mutationen Q161M, S169V, D196N, A220L, K283A, M633I, L705T und L788V auf.

Insbesondere die Aminosäure-Sequenzen gemäß SEQ ID NO:19 und SEQ ID NO:21 zeichnen sich im Vergleich zur Aminosäure-Sequenz gemäß SEQ ID NO:1 im Hinblick auf die Katalyse der Synthese von Cellobiose aus Glukose-1-Phosphat und Glukose durch folgende Eigenschaften aus:
(i) erhöhte spezifische Aktivität für die Umwandlung von Glukose und Glukose-1-Phosphat insbesondere bei höheren Substratkonzentrationen, während die Aktivität im Hinblick auf die phosphorolytische Umkehrreaktion weitgehend erhalten bleibt; und/oder
(ii) erhöhte Raum-Zeit-Ausbeute pro eingesetzte Enzymmenge, insbesondere bei erhöhter Konzentration an Glukose aufgrund einer verbesserten Edukt- und Produkt-Toleranz bzw. einer geringeren Edukt- und Produkt-Inhibition.

Dabei bedeutet die Eigenschaft unter (i), dass im Vergleich zur Aminosäure-Sequenz gemäß gemäß SEQ ID NO:1 ein erhöhtes Synthese/Phosphorolyse-Verhältnis erreicht wird.

Dabei bedeutet die Eigenschaft unter (ii), dass im Vergleich zur Aminosäure-Sequenz gemäß gemäß SEQ ID NO:1 eine erhöhte Raum-Zeit-Ausbeute pro eingesetzter Enzymmenge erreicht wird. Die Raum-Zeit-Ausbeute ist definiert als die pro Volumen und Zeit gebildete Produktmenge bei maximalem Substrat-Umsatz.

Die Aminosäure-Sequenz gemäß SEQ ID NO:8, SEQ ID NO:18 und SEQ ID NO:19 zeichnen sich im Vergleich zur Aminosäure-Sequenz gemäß SEQ ID NO:1 zudem durch eine verbesserte Temperaturstabilität aus.

In nachfolgender Tabelle sind die Unterschiede dieser erfindungsgemäßen Aminosäure-Sequenzen im Vergleich zur Cellobiose-Phosphorylase aus *Cellulomonas uda* (Wildtyp, SEQ ID NO:1) einander gegenübergestellt:

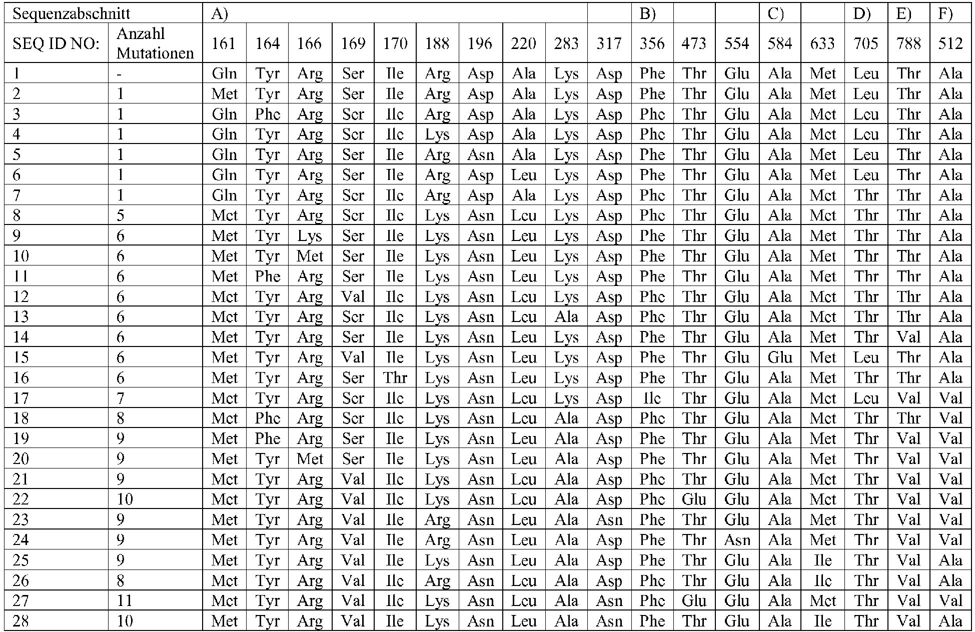

Bevorzugt katalysiert die erfindungsgemäße Cellobiose-Phosphorylase die Umsetzung von Glukose-1-Phosphat zu Cellobiose und/oder die Umsetzung von Glukose-1-Phosphat mit Glukose zu Cellobiose.

Es wurde überraschend gefunden, dass die erfindungsgemäße Cellobiose-Phosphorylase im Vergleich zur Cellobiose-Phosphorylase gemäß SEQ ID NO:1 im Hinblick auf die Umsetzung von Glukose-1-Phosphat mit Glukose zu Cellobiose Vorteile aufweist, insbesondere
(i) eine gesteigerte Syntheseaktivität in Gegenwart äquimolarer Mengen Glukose und Glukose-1-Phosphat von 250, 500 und 750 mM und/oder
(ii) eine höhere Raum-Zeit-Ausbeute pro eingesetzter Enzymmenge in Gegenwart äquimolarer Mengen Glukose und Glukose-1-Phosphat von 250 und 500 mM; und/oder
(iii) ein erhöhtes Synthese/Phosphorolyse-Verhältnis, welches bevorzugt mindestens 2fach erhöht, bevorzugter mindestens 4fach, mindestens 6fach, mindestens 8fach, mindestens 10fach, mindestens 15fach, mindestens 20fach oder mindestens 25fach erhöht ist.

Ferner wurde überraschend gefunden, dass die erfindungsgemäße Cellobiose-Phosphorylase im Vergleich zur Cellobiose-Phosphorylase gemäß SEQ ID NO:1 im Hinblick auf die Prozessstabilität Vorteile haben kann, insbesondere eine höhere Temperaturstabilität, z.B. nach Inkubation bei 58°C für 15 min.

Ein weiterer Aspekt der Erfindung betrifft die Verwendung der in den Ansprüchen beschriebenen, erfindungsgenäßen Cellobiose-Phosphorylase zur enzymatisch katalysierten Umsetzung von Glukose-1-Phosphat zu Cellobiose und/oder von Glukose-1-Phosphat mit Glukose zu Cellobiose.

Ein weiterer Aspekt der Erfindung betrifft ein Verfahren zur Herstellung von Cellobiose umfassend die Umsetzung von Glukose-1-Phosphat und ggf. von Glukose unter enzymatischer Katalyse durch die in den Ansprüchen beschriebene, erfindungsgemäße Cellobiose-Phosphorylase.

Bevorzugt umfasst das offenbarte Verfahren die Schritte
(a) Synthese von Glukose-1-Phosphat und Fructose durch Umsetzen von Saccharose und Phosphat unter enzymatischer Katalyse durch eine Saccharose-Phosphorylase;
(b) Synthese von Cellobiose und Phosphat durch Umsetzen des Glukose-1-Phosphats mit Glukose unter enzymatischer Katalyse durch die Cellobiose-Phosphorylase.

Die nachfolgenden Beispiele illustrieren die Erfindung, sind jedoch nicht einschränkend auszulegen:

### Definitionen, Material und Methoden

### Phosphorolyse-Units (PU):

Eine Phosphorylase-Unit (=PU) ist definiert als die Menge an Enzym, die in einer Minute bei 30°C in Gegenwart von 10 mM Cellobiose und 75 mM Kaliumphosphatpuffer pH7, 1 µmol α-D-Glucose 1-Phosphat bildet. Die Bestimmung der Phosphorylase-Aktivität (PU) wurde über den Nachweis des in der Cellobiose Phosphorylase Reaktion gebildeten α-D-Glucose-1-Phosphats mit einer gekoppelten Reaktion aus Phosphoglucomutase und Glucose-6-Phosphat-Dehydrogenase gemessen. Phosphoglucomutase setzt α-D-Glucose-1-Phosphat zu Glucose 6-Phosphat um, welches unter Bildung von NADPH zu 6-Phosphogluconat katalysiert durch eine Glucose-6-Phosphat-Dehydrogenase reagiert. Die Bildung von NADPH kann über die Veränderung der Absorption bei 340 nm quantifiziert werden. Die Bildung eines mols NADPH korreliert mit der Bildung eines mols α-D-Glucose-1-Phosphat.

### HPLC-Messung Glucose und Cellobiose:

Der Nachweis von Glucose und Cellobiose erfolgte per HPLC. Eine Trennung der Analyten wurde bei einer Flussrate von 2 mL/min, bei 40°C auf einer 250x4.6 Luna-NH2 5µm 100Å (Phenomenex) erreicht. Als Laufmittel wurde ein Acetonitril-Wasser Gemisch im Verhältnis 79/21 (v/v) verwendet. Die Detektion der Analyten erfolgte mit Hilfe eines RID-Detektor.

### Herstellung eines Cellobiose Phosphorylase-haltigen Zellrohextraktes:

Die Cellobiose-Phosphorylasen gemäß SEQ ID NO:1-28 wurden auf einem rekombinanten Plasmid in *E. coli* BL21(DE3) exprimiert. Ein Schüttelkolben mit 20 mL eines für *E. coli* üblichen Kultivierungsmediums wie bspw. LB- oder TB-Medium mit 50 µg/mL Kanamycin wurde mit einer Übernachtkultur auf eine OD₆₀₀ von 0,1 angeimpft und bis zu einer OD₆₀₀ von 0,6-0,8 bei 37°C und 200 rpm inkubiert. Dann wurde c_{final}= 0,1 mM IPTG zugegeben und die Kultur bei 30°C und 200 rpm über Nacht inkubiert. Die Kultur wurde über Zentrifugation geerntet und in einem Aufschlusspuffer bestehend aus 50 mM Kaliumphosphatpuffer pH 7, 10 mM MgCl₂, 0,5 mg/mL Lysozym und 20 U/mL eines Nuclease-Enzym wie bspw. Benzonase aufgenommen. Der Zellaufschluss erfolgte mit Ultraschall. Unlösliche Bestandteile wurden über Zentrifugation abgetrennt und das so erhaltene Cellobiose-Phosphorylase-haltige Rohextrakt in den folgend beschriebenen Beispielen eingesetzt.

### Ausführungsbeispiele

### Beispiel 1 (Phosphorolyse-Aktivität):

Die Phosphorolyse-Aktivität von Cellobiose Phosphorylase-haltigen Zellrohextrakten des Enzyms gemäß SEQ ID NO:1, 8, 18, 19, 20 und 21 wurde bestimmt und auf eine Zelldichte von OD₆₀₀=1 normiert. Die Ergebnisse sind in Tabelle 1 dargestellt. Die Phosphorolyse-Aktivitätsausbeuten pro Zelle der Varianten gemäß SEQ ID NO:8, 18, 19, 20 und 21 lagen zwischen 69% und 127% im Vergleich zu SEQ ID NO:1.

**Tabelle 1:**

| SEQ ID NO: | PU/OD₆₀₀=1 |
|---|---|
| 1 | 1,12 |
| 8 | 0,96 |
| 18 | 1,35 |
| 19 | 1,42 |
| 20 | 0,77 |
| 21 | 0,90 |

### Beispiel 2 (Synthese zu Phosphorolyse-Verhältnis, 250mM Susbtrate):

In einem 0,5 mL Reaktionsgefäß wurde 50 µL eines Cellobiose-Phosphorylase-haltigen Rohextraktes des Enzyms gemäß SEQ ID NO:1, 8, 18, 19, 20 und 21 vorgelegt. Dazu wurde 150 µL einer auf 30°C vortemperierten Substratlösung mit äquimolar 333,3 mM α-D-Glucose 1-Phosphat und D-Glucose in 50 mM MES-Puffer pH 6,5 zugegeben. α-D-Glucose 1-Phosphat kann aus der Reaktion einer Saccharose Phosphorylase mit Saccharose und Phosphat stammen oder als Reinsubstanz eingesetzt werden. Die Ansätze wurden 30 min bei 30°C und 300 rpm und durch eine Inkubation für 10 min bei 98°C inaktiviert. Die Ansätze wurden zentrifugiert und der Cellobiose-Gehalt per HPLC bestimmt. Eine Synthese-Unit bei 250 mM Substrat (=SU (250mM)) ist die Menge an Enzym, die in einer Minute bei 30°C in Gegenwart von 250 mM α-D-Glucose-1-Phosphat und 250 mM D-Glucose in 50 mM MES-Puffer pH 6,5, 1 µmol Cellobiose bildet. Die Ergebnisse sind in Tabelle 2 dargestellt. Die Varianten gemäß SEQ ID NO:18, 19, 20, und 21 zeigen eine mehr als 2-fache Steigerung im Vergleich zum Wildtypenzym.

**Tabelle 2:**

| SEQ ID NO: | SU(250mM)/PU | Faktor zu SEQ ID NO:1 |
|---|---|---|
| 1 | 0,10 | 1,0 |
| 8 | 0,08 | 0,8 |
| 18 | 0,24 | 2,4 |
| 19 | 0,24 | 2,4 |
| 20 | 0,79 | 7,8 |
| 21 | 1,60 | 15,9 |

### Beispiel 3 (Synthese zu Phosphorolyse-Verhältnis, 500 mM Substrate):

Beispiel 2 wurde wiederholt mit einer Substratlösung bestehend aus äquimolar 666,6 mM α-D-Glucose 1-Phosphat und D-Glucose in 50 mM MES-Puffer pH 6,5. α-D-Glucose 1-Phosphat kann aus der Reaktion einer Saccharose Phosphorylase mit Saccharose und Phosphat stammen oder als Reinsubstanz eingesetzt werden. Die Ergebnisse sind in Tabelle 3 dargestellt.. Eine Synthese-Unit bei 500 mM Substrat (=SU (500 mM)) ist die Menge an Enzym, die in einer Minute bei 30°C in Gegenwart von 500 mM α-D-Glucose-1-Phosphat und 500 mM D-Glucose in 50 mM MES-Puffer pH 6,5, 1 µmol Cellobiose bildet. Bei einer Erhöhung der Substratkonzentration auf 500 mM zeigen alle Varianten eine noch höhere Steigerung im Vergleich zur Cellobiose Phosphorylase gemäß SEQ ID NO:1. Die Verbesserung der besten Variante erreicht eine Steigerung um den Faktor 28.

**Tabelle 3:**

| SEQ ID NO: | SU(500mM)/PU | Faktor zu SEQ ID NO:1 |
|---|---|---|
| 1 | 0,04 | 1,0 |
| 8 | 0,05 | 1,1 |
| 18 | 0,13 | 2,9 |
| 19 | 0,15 | 3,4 |
| 20 | 0,71 | 15,8 |
| 21 | 1,27 | 28,2 |

### Beispiel 4 (Synthese zu Phosphorolyse-Verhältnis, 750 mM Substrate):

Beispiel 2 wurde wiederholt mit einer Substratlösung bestehend aus äquimolar 1000 mM α-D-Glucose 1-Phosphat und D-Glucose in 50 mM MES-Puffer pH6,5 . α-D-Glucose 1-Phosphat kann aus der Reaktion einer Saccharose Phosphorylase mit Saccharose und Phosphat stammen oder als Reinsubstanz eingesetzt werden. Die Ergebnisse sind in Tabelle 4 dargestellt.. Eine Synthese-Unit bei 750 mM Substrat (=SU (750mM)) ist die Menge an Enzym, die in einer Minute bei 30°C in Gegenwart von 750 mM α-D-Glucose-1-Phosphat und 750 mM D-Glucose in 50 mM MES-Puffer pH6,5, 1 µmol Cellobiose bildet. Mit der Cellobiose Phosphorylase gemäß SEQ ID NO:1 und 8 konnte keine Cellobiose-Bildung beobachtet werden. Im Gegensatz dazu, erreichten die Varianten gemäß SEQ ID NO:18, 19, 20 und 21 deutliche Cellobiose-Bildung und somit eine Steigerung zur Cellobiose Phosphorylase gemäß SEQ ID NO:1 um mind. 2,5, 3,6, 17,5, bzw. 29,6.

**Tabelle 4:**

| SEQ ID NO: | SU(750mM)/PU | Faktor zu SEQ ID NO:1 |
|---|---|---|
| 1 | unterhalb der Nachweisgrenze (<0,03 SU(750mM)/PU) | -- |
| 8 | unterhalb der Nachweisgrenze (<0,03 SU(750mM)/PU) | -- |
| 18 | 0,08 | > 2,5 |
| 19 | 0,11 | > 3,6 |
| 20 | 0,53 | > 17,5 |
| 21 | 0,89 | > 29,6 |

### Beispiel 5 (Temperaturstabilität):

50 µL Cellobiose-Phosphorylase-haltiges Rohextraktes der Cellobiose Phosphorylase gemäß SEQ ID NO:1, 8, 18 und 19 wurden in eine PCR-Mikrotiterplatte überführt und in einem Gradienten PCR-Cycler für 15 min bei Temperaturen zwischen 50,2 und 62,5 °C inkubiert. Präzipitiertes Protein wurde über Zentrifugation abgetrennt und die Phosphorolyse-Aktivität des Überstands bestimmt. In Tabelle 5 sind die erhaltenen Restaktivitäten aufgelistet. Die Restaktivität der Cellobiose Phsophorylase gemäß SEQ ID NO:1 sinkt nach Inkubation bei 58,1 °C auf 2%. Die Varianten gemäß SEQ ID NO:8, 18 und 19 hingegen zeigten eine deutlich höhere Temperaturstabilität und besitzen nach Inkubation bei 58,1 °C noch zwischen 15,4 und 55,8% Restaktivität.

**Tabelle 5:**

| Temperaturbehandlung [°C] | Restaktivität [%] | | | |
|---|---|---|---|---|
| | SEQ ID NO:1 | SEQ ID NO:8 | SEQ ID NO:18 | SEQ ID NO:19 |
| ohne Temperaturbehandlung | 100,0 | 100,0 | 100,0 | 100,0 |
| 50,2 | 82,4 | 101,9 | 98,0 | 89,5 |
| 50,8 | | 102,0 | | 85,7 |
| 50,9 | | | 92,5 | |
| 51 | 76,8 | | | |
| 51,9 | | 96,4 | 93,5 | 82,7 |
| 52,4 | 63,2 | | | |
| 53,3 | | 94,4 | 80,9 | 77,4 |
| 54,1 | 29,7 | | | |
| 54,8 | | 86,9 | 64,4 | 56,3 |
| 56,1 | 4,0 | | | |
| 56,5 | | 77,6 | 41,6 | 37,2 |
| 58,1 | 2,0 | 55,8 | 19,6 | 15,4 |
| 59,7 | | 21,3 | 1,1 | 0,6 |
| 60,2 | 0,9 | | | |
| 61 | | 6,0 | 0,0 | 0,0 |
| 61,9 | | 1,1 | 0,0 | 0,0 |
| 62,1 | 0,2 | | | |
| 62,5 | | 0,8 | 0,0 | 0,0 |

### Beispiel 6 (Langzeitaktivität bei 30°C):

400 µL Cellobiose-Phosphorylase-haltiges Rohextrakt der Cellobiose Phosphorylasen gemäß SEQ ID NO:8, 18 und 19 wurden in ein 1,5 mL Reaktionsgefäß überführt und in einem Brutschrank bei 30°C inkubiert. Die Phosphorolyse-Aktivität wurde über einen Zeitraum von 28 Tagen getestet. Die Ergebnisse sind in Tabelle 6 dargestellt. Alle Varianten besitzen nach Inkubation über 28 Tagen bei 30°C noch eine Aktivität von >60%.

**Tabelle 6:**

| Inkubationsdauer bei 30°C [d] | Restaktivität [%] | | |
|---|---|---|---|
| | SEQ ID NO:8 | SEQ ID NO:18 | SEQ ID NO:19 |
| 0 | 100 | 100 | 100 |
| 6 | 94 | 93 | 98 |
| 13 | 85 | 86 | 89 |
| 19 | 72 | 78 | 78 |
| 28 | 64 | 75 | 67 |

### Beispiel 7 (Cellobiose Synthese mit 250 mM Substraten):

In einem 2 mL Reaktionsgefäß wurde 300 µL eines Cellobiose-Phosphorylase-haltigen Rohextraktes der Varianten gemäß SEQ ID NO:8, 19, 20 und 21 vorgelegt. Dazu wurden 1,7 mL einer auf 30°C vortemperierten Substratlösung mit äquimolar 294,1 mM α-D-Glucose 1-Phosphat und D-Glucose in 50 mM MES-Puffer pH6,5 zugegeben. Die Ansätze wurden bei 30°C und 300 rpm inkubiert. Bis zu Erreichen des maximalen Umsatzes wurde über einen Zeitraum von 45,9h Proben entnommen und durch eine Inkubation für 10 min bei 98°C inaktiviert. Die Ansätze wurden zentrifugiert und der Cellobiose-Gehalt per HPLC bestimmt. Die Variante gemäß SEQ ID NO:21 erreichte die höchste Raum-Zeitausbeute pro eingesetzter kPU von 19,6 mmol(Cellobiose)/(h*L*kPU).

**Tabelle 7:**

| SEQ ID NO: | mmol(Cellobiose)/(h*L*kPU) |
|---|---|
| 8 | 1,11 |
| 19 | 5,0 |
| 20 | 20,0 |
| 21 | 19,6 |

### Beispiel 8 (Cellobiose Synthese mit 500 mM Substraten):

Beispiel 7 wurde wiederholt mit einer Substratlösung mit äquimolar 588,2 mM α-D-Glucose 1-Phosphat und D-Glucose in 50 mM MES-Puffer pH6,5. Bei dieser höheren Substratkonzentration erreichte die Variante gemäß SEQ ID NO:21 eine noch höhere Raum-Zeitausbeute pro eingesetzter kPU von 25,3 mmol(Cellobiose)/(h*L*kPU).

**Tabelle 8:**

| SEQ ID NO: | mmol(Cellobiose)/(h*L*kPU) |
|---|---|
| 8 | 0,5 |
| 19 | 3,5 |
| 20 | 18,0 |
| 21 | 25,3 |

### Beispiel 9 (Cellobiose Synthese aus Saccharose und Glukose unter Einsatz einer Saccharose-Phosphorylase und der erfindungsgemäßen Cellobiose-Phosphorylase):

256 g Saccharose werden mit 48 g Natriumdihydrogenphosphat (NaH₂PO₄) und 57 g Dinatriumhydrogenphosphat (Na₂HPO₄) oder 70 g Dikaliumhydrogenphosphat (K₂HPO₄) und 48 g Kaliumdihydrogenphosphat (KH₂PO₄) in 1 L Wasser gelöst. Die Prozesslösung wird in einem Rührkesselreaktor auf eine Reaktionstemperatur von 30 °C gebracht und die Reaktion durch Zugabe von 5 kU Saccharose-Phosphorylase aus *Bifidobacterium adolescentis* gestartet. Der pH-Wert der Prozesslösung liegt bei 6,5 und wird ggf. mit Phosphorsäure oder Natrium-oder Kaliumhydroxid korrigiert. Die Reaktion läuft über 20 h. Die Reaktionslösung wird über eine PES(Polyethersulfon)-Membran mit einem Cut-Off von 10 kDa ultrafiltriert. Das Permeat wird equimolar zu Glukose-1-Phosphat mit Glukose als Reaktionspartner in einem Rührkesselreaktor versetzt und auf Reaktionstemperatur von 30 °C gebracht (Endkonzonzentration Glukose-1-Phosphat in erster Produktzusammensetzung: ca. 0,5 M. Die Reaktion wird durch die Zugabe von 3kU (Phosphorylaseaktivität) einer Cellobiose-Phosphorylase aus *Cellulomonas uda* (entweder die Mutante SEQ-ID No: 8 oder SEQ-ID No: 19 oder SEQ-ID No: 21) versetzt. Die Reaktion läuft bei pH 6,5 über 24 h.

**Tabelle 9:**

| Enzym SEQ-ID No: | Cellobiose [g/L] |
|---|---|
| 8 | 31 |
| 19 | 41 |
| 21 | 124 |

## Patentansprüche

1. Cellobiose-Phosphorylase umfassend eine Aminosäuresequenz, welche eine Identität zur Aminosäuresequenz gemäß SEQ ID NO:21 von mindestens 90% aufweist;
wobei die Aminosäure-Sequenz im Vergleich zu SEQ ID NO:1 eine oder mehrere Aminosäure-Mutationen in Positionen 161, 169, 196, 220, 283, 512, 705 aufweist; und
wobei die Aminosäure-Sequenz keine Aminosäure-Mutation K283H umfasst; und
wobei die Cellobiose-Phosphorylase im Vergleich zur Cellobiose-Phosphorylase gemäß SEQ ID NO: 1
(i) im Hinblick auf die Umsetzung von Glukose-1-Phosphat mit Glukose zu Cellobiose eine gesteigerte Syntheseaktivität in Gegenwart äquimolarer Mengen Glukose und Glukose-1-Phosphat von 250, 500 und 750 mM; und/oder
(ii) im Hinblick auf die Umsetzung von Glukose-1-Phosphat mit Glukose zu Cellobiose eine höhere Raum-Zeit-Ausbeute pro eingesetzter Enzymmenge in Gegenwart äquimolarer Mengen Glukose und Glukose-1-Phosphat von 250 und 500 mM; und/oder
(iii) eine höhere Temperaturstabilität nach Inkubation bei 58 °C für 15 min
aufweist.

2. Die Cellobiose-Phosphorylase nach Anspruch 1, welche eine Identität zur Aminosäuresequenz gemäß SEQ ID NO:21 von mindestens 91% aufweist.

3. Die Cellobiose-Phosphorylase nach Anspruch 2, welche eine Identität zur Aminosäuresequenz gemäß SEQ ID NO:21 von mindestens 92% aufweist.

4. Die Cellobiose-Phosphorylase nach Anspruch 3, welche eine Identität zur Aminosäuresequenz gemäß SEQ ID NO:21 von mindestens 93% aufweist.

5. Die Cellobiose-Phosphorylase nach Anspruch 4, welche eine Identität zur Aminosäuresequenz gemäß SEQ ID NO:21 von mindestens 94% aufweist.

6. Die Cellobiose-Phosphorylase nach Anspruch 5, welche eine Identität zur Aminosäuresequenz gemäß SEQ ID NO:21 von mindestens 95% aufweist.

7. Die Cellobiose-Phosphorylase nach Anspruch 6, welche eine Identität zur Aminosäuresequenz gemäß SEQ ID NO:21 von mindestens 96% aufweist.

8. Die Cellobiose-Phosphorylase nach Anspruch 7, welche eine Identität zur Aminosäuresequenz gemäß SEQ ID NO:21 von mindestens 97% aufweist.

9. Die Cellobiose-Phosphorylase nach Anspruch 8, welche eine Identität zur Aminosäuresequenz gemäß SEQ ID NO:21 von mindestens 98% aufweist.

10. Die Cellobiose-Phosphorylase nach einem der vorstehenden Ansprüche, wobei die Aminosäure-Sequenz im Vergleich zu SEQ ID NO:1 eine oder mehrere der Aminosäure-Mutationen Q161M, S169V, R188K, D196N, A220L, K283A, A512V, L705T und T788V aufweist.

11. Die Cellobiose-Phosphorylase nach einem der vorstehenden Ansprüche, welche die Umsetzung von Glukose-1-Phosphat zu Cellobiose und/oder die Umsetzung von Glukose-1-Phosphat mit Glukose zu Cellobiose katalysiert.

12. Verwendung der Cellobiose-Phosphorylase nach einem der Ansprüche 1 bis 11 zur enzymatisch katalysierten Umsetzung von Glukose-1-Phosphat zu Cellobiose und/oder von Glukose-1-Phosphat mit Glukose zu Cellobiose.

13. Ein Verfahren zur Herstellung von Cellobiose umfassend die Umsetzung von Glukose-1-Phosphat und ggf. von Glukose unter enzymatischer Katalyse durch die Cellobiose-Phosphorylase nach einem der Ansprüche 1 bis 11.

14. Das Verfahren nach Anspruch 13, umfassend die Schritte
(a) Synthese von Glukose-1-Phosphat und Fructose durch Umsetzen von Saccharose und Phosphat unter enzymatischer Katalyse durch eine Saccharose-Phosphorylase;
(b) Synthese von Cellobiose und Phosphat durch Umsetzen des Glukose-1-Phosphats mit Glukose unter enzymatischer Katalyse durch die Cellobiose-Phosphorylase.

## Claims

1. Cellobiose phosphorylase comprising an amino acid sequence having an identity of at least 90% in relation to the amino acid sequence according to SEQ ID NO:21;
the amino acid sequence having, in comparison with SEQ ID NO:1, one or more amino acid mutations in positions 161, 169, 196, 220, 283, 512 and 705; and
the amino acid sequence comprising no K283H amino acid mutation; and
the cellobiose phosphorylase having
(i) with respect to the reaction of glucose 1-phosphate with glucose to form cellobiose, an increased synthesis activity in the presence of equimolar amounts of glucose and glucose 1-phosphate of 250, 500 and 750 mM; and/or
(ii) with respect to the reaction of glucose 1-phosphate with glucose to form cellobiose, a higher space-time yield per enzyme amount used in the presence of equimolar amounts of glucose and glucose 1-phosphate of 250 and 500 mM; and/or
(iii) a higher temperature stability after incubation at 58°C for 15 min
in comparison with the cellobiose phosphorylase according to SEQ ID NO:1.

2. Cellobiose phosphorylase according to Claim 1, which has an identity of at least 91% in relation to the amino acid sequence according to SEQ ID NO:21.

3. Cellobiose phosphorylase according to Claim 2, which has an identity of at least 92% in relation to the amino acid sequence according to SEQ ID NO:21.

4. Cellobiose phosphorylase according to Claim 3, which has an identity of at least 93% in relation to the amino acid sequence according to SEQ ID NO:21.

5. Cellobiose phosphorylase according to Claim 4, which has an identity of at least 94% in relation to the amino acid sequence according to SEQ ID NO:21.

6. Cellobiose phosphorylase according to Claim 5, which has an identity of at least 95% in relation to the amino acid sequence according to SEQ ID NO:21.

7. Cellobiose phosphorylase according to Claim 6, which has an identity of at least 96% in relation to the amino acid sequence according to SEQ ID NO:21.

8. Cellobiose phosphorylase according to Claim 7, which has an identity of at least 97% in relation to the amino acid sequence according to SEQ ID NO:21.

9. Cellobiose phosphorylase according to Claim 8, which has an identity of at least 98% in relation to the amino acid sequence according to SEQ ID NO:21.

10. Cellobiose phosphorylase according to any of the preceding claims, wherein the amino acid sequence has, in comparison with SEQ ID NO:1, one or more of the amino acid mutations Q161M, S169V, R188K, D196N, A220L, K283A, A512V, L705T and T788V.

11. Cellobiose phosphorylase according to any of the preceding claims, which catalyses the conversion of glucose 1-phosphate to form cellobiose and/or the reaction of glucose 1-phosphate with glucose to form cellobiose.

12. Use of the cellobiose phosphorylase according to any of Claims 1 to 11 for the enzymatically catalysed conversion of glucose 1-phosphate to form cellobiose and/or enzymatically catalysed reaction of glucose 1-phosphate with glucose to form cellobiose.

13. Method for producing cellobiose, comprising the conversion of glucose 1-phosphate and optionally of glucose under enzymatic catalysis by the cellobiose phosphorylase according to any of Claims 1 to 11.

14. Method according to Claim 13, comprising the steps of
(a) synthesizing glucose 1-phosphate and fructose by conversion of sucrose and phosphate under enzymatic catalysis by a sucrose phosphorylase;
(b) synthesizing cellobiose and phosphate by reaction of the glucose 1-phosphate with glucose under enzymatic catalysis by the cellobiose phosphorylase.

## Revendications

1. Cellobiose phosphorylase comprenant une séquence d'acides aminés qui présente une identité avec la séquence d'acides aminés selon SEQ ID NO : 21 d'au moins 90 % ;
la séquence d'acides aminés présentant en comparaison de SEQ ID NO : 1 une ou plusieurs mutations d'acides aminés aux positions 161, 169, 196, 220, 283, 512, 705 ; et
la séquence d'acides aminés ne comprenant pas de mutation d'acides aminés K283H ; et
la cellobiose phosphorylase présentant en comparaison de la cellobiose phosphorylase selon SEQ ID NO : 1 :
(i) au regard de la réaction de glucose-1-phosphase avec du glucose pour former de la cellobiose, une activité de synthèse augmentée en présence de quantités équimolaires de glucose et de glucose-1-phosphate de 250, 500 et 750 mM ; et/ou
(ii) au regard de la réaction de glucose-1-phosphate avec du glucose pour former de la cellobiose, un rendement espace-temps plus élevé par quantité d'enzyme utilisée en présence de quantités équimolaires de glucose et de glucose-1-phosphate de 250 et 500 mM ; et/ou
(iii) une stabilité à la température plus élevée après une incubation à 58 °C pendant 15 minutes.

2. Cellobiose phosphorylase selon la revendication 1, qui présente une identité avec la séquence d'acides aminés selon SEQ ID NO : 21 d'au moins 91 %.

3. Cellobiose phosphorylase selon la revendication 2, qui présente une identité avec la séquence d'acides aminés selon SEQ ID NO : 21 d'au moins 92 %.

4. Cellobiose phosphorylase selon la revendication 3, qui présente une identité avec la séquence d'acides aminés selon SEQ ID NO : 21 d'au moins 93 %.

5. Cellobiose phosphorylase selon la revendication 4, qui présente une identité avec la séquence d'acides aminés selon SEQ ID NO : 21 d'au moins 94 %.

6. Cellobiose phosphorylase selon la revendication 5, qui présente une identité avec la séquence d'acides aminés selon SEQ ID NO : 21 d'au moins 95 %.

7. Cellobiose phosphorylase selon la revendication 6, qui présente une identité avec la séquence d'acides aminés selon SEQ ID NO : 21 d'au moins 96 %.

8. Cellobiose phosphorylase selon la revendication 7, qui présente une identité avec la séquence d'acides aminés selon SEQ ID NO : 21 d'au moins 97 %.

9. Cellobiose phosphorylase selon la revendication 8, qui présente une identité avec la séquence d'acides aminés selon SEQ ID NO : 21 d'au moins 98 %.

10. Cellobiose phosphorylase selon l'une quelconque des revendications précédentes, dans laquelle la séquence d'acides aminés présente en comparaison de SEQ ID NO : 1 une ou plusieurs des mutations d'acides aminés Q161M, S169V, R188K, D196N, A220L, K283A, A512V, L705T et T788V.

11. Cellobiose phosphorylase selon l'une quelconque des revendications précédentes, qui catalyse la réaction de glucose-1-phosphate pour former de la cellobiose et/ou la réaction de glucose-1-phosphate avec du glucose pour former de la cellobiose.

12. Utilisation de la cellobiose phosphorylase selon l'une quelconque des revendications 1 à 11 pour la réaction sous catalyse enzymatique de glucose-1-phosphate pour former de la cellobiose et/ou de glucose-1-phosphate avec du glucose pour former de la cellobiose.

13. Procédé de fabrication de cellobiose comprenant la réaction de glucose-1-phosphate et éventuellement de glucose sous catalyse enzymatique par la cellobiose phosphorylase selon l'une quelconque des revendications 1 à 11.

14. Procédé selon la revendication 13, comprenant les étapes suivantes :
(a) la synthèse de glucose-1-phosphate et de fructose par mise en réaction de saccharose et de phosphate sous catalyse enzymatique par une saccharose phosphorylase ;
(b) la synthèse de cellobiose et de phosphate par mise en réaction du glucose-1-phosphate avec du glucose sous catalyse enzymatique par la cellobiose phosphorylase.


### SEQUENCE LISTING

<110> Pfeifer & Langen GmbH & Co. KG
<120> Cellobiose-Phosphorylase
<130> PFL0002-WO
<150> EP14184301
   <151> 2014-09-10
<150> EP14190891
   <151> 2014-10-29
<160> 28
<170> PatentIn version 3.5
<210> 1
   <211> 822
   <212> PRT
   <213> Cellulomonas uda
<400> 1
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Gln Thr Asn Tyr Gln Arg Asn Leu Ser Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asp His Tyr Ala Val Phe Ala Val Asn Thr Gln Ala Glu
           195                 200                 205
   Gly Phe Asp Thr Asp Arg Asp Thr Phe Val Gly Ala Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Leu Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 2
   <211> 822
   <212> PRT
   <213> artificial
<220>
   <223> modified wildtye sequence
<400> 2
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Ser Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asp His Tyr Ala Val Phe Ala Val Asn Thr Gln Ala Glu
           195                 200                 205
   Gly Phe Asp Thr Asp Arg Asp Thr Phe Val Gly Ala Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Leu Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 3
   <211> 822
   <212> PRT
   <213> artificial
<220>
   <223> modified wildtye sequence
<400> 3
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Gln Thr Asn Phe Gln Arg Asn Leu Ser Ile Gly Glu Val Glu Val Glu
                   165                 170                 175
   Gln Glu Ser Pro His Gly Ser Ala Ile Tyr His Arg Thr Glu Tyr Arg
   180 185 190
   Glu Arg Arg Asp His Tyr Ala Val Phe Ala Val Asn Thr Gln Ala Glu
   195 200 205
   Gly Phe Asp Thr Asp Arg Asp Thr Phe Val Gly Ala Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Leu Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Thr Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 4
   <211> 822
   <212> PRT
   <213> artificial
<220>
   <223> modified wildtye sequence
<400> 4
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Gln Thr Asn Tyr Gln Arg Asn Leu Ser Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asp His Tyr Ala Val Phe Ala Val Asn Thr Gln Ala Glu
   195 200 205
   Gly Phe Asp Thr Asp Arg Asp Thr Phe Val Gly Ala Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Leu Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
   820
<210> 5
   <211> 822
   <212> PRT
   <213> artificial
<220>
   <223> modified wildtye sequence
<400> 5
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Gln Thr Asn Tyr Gln Arg Asn Leu Ser Ile Gly Glu Val Glu Val Glu
   165 170 175
   Gln Glu Ser Pro His Gly Ser Ala Ile Tyr His Arg Thr Glu Tyr Arg
   180 185 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Ala Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Leu Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 6
   <211> 822
   <212> PRT
   <213> artificial
<220>
   <223> modified wildtye sequence
<400> 6
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Gln Thr Asn Tyr Gln Arg Asn Leu Ser Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asp His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Leu Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Thr Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 7
   <211> 822
   <212> PRT
   <213> artificial
<220>
   <223> modified wildtye sequence
<400> 7
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Gln Thr Asn Tyr Gln Arg Asn Leu Ser Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asp His Tyr Ala Val Phe Ala Val Asn Thr Gln Ala Glu
           195                 200                 205
   Gly Phe Asp Thr Asp Arg Asp Thr Phe Val Gly Ala Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 8
   <211> 822
   <212> PRT
   <213> artificial
<220>
   <223> modified wildtye sequence
<400> 8
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Arg Asn Leu Ser Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 9
   <211> 822
   <212> PRT
   <213> artificial
<220>
   <223> modified wildtye sequence
<400> 9
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Lys Asn Leu Ser Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 10
   <211> 822
   <212> PRT
   <213> artificial
<220>
   <223> modified wildtye sequence
<400> 10
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Met Asn Leu Ser Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Thr Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 11
   <211> 822
   <212> PRT
   <213> artificial
<220>
   <223> modified wildtye sequence
<400> 11
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Phe Gln Arg Asn Leu Ser Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 12
   <211> 822
   <212> PRT
   <213> artificial
<220>
   <223> modified wildtye sequence
<400> 12
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145 150 155 160
   Met Thr Asn Tyr Gln Arg Asn Leu Ser Val Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Lys Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Thr Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 13
   <211> 822
   <212> PRT
   <213> artificial
<220>
   <223> modified wildtye sequence
<400> 13
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Arg Asn Leu Ser Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 14
   <211> 822
   <212> PRT
   <213> artificial
<220>
   <223> modified wildtye sequence
<400> 14
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Arg Asn Leu Ser Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Ala
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
                   660                 665                 670
   Ile Ala Glu Thr Val Val Gly Arg Gly Gly Arg Ala Phe Asp Tyr Tyr
               675                 680                 685
   Lys Arg Ile Thr Pro Ala Tyr Arg Glu Asp Ile Ser Asp Val His Arg
           690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Val Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 15
   <211> 822
   <212> PRT
   <213> artificial
<220>
   <223> modified wildtye sequence
<400> 15
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Glu His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Leu Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
   805 810 815
   Arg Val Asp Val Thr Leu
   820
<210> 16
   <211> 822
   <212> PRT
   <213> artificial
<220>
   <223> modified wildtye sequence
<400> 16
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Arg Asn Leu Ser Thr Gly Glu Val Glu Val Glu
   165 170 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Thr Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 17
   <211> 822
   <212> PRT
   <213> artificial
<220>
   <223> modified wildtye sequence
<400> 17
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Ser Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Lys Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Ile Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Val
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Leu Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 18
   <211> 822
   <212> PRT
   <213> artificial
<220>
   <223> modified wildtye sequence
<400> 18
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Phe Gln Arg Asn Leu Ser Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Val
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Thr Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 19
   <211> 822
   <212> PRT
   <213> artificial
<220>
   <223> modified wildtye sequence
<400> 19
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Phe Gln Arg Asn Leu Ser Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Val
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Val Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 20
   <211> 822
   <212> PRT
   <213> artificial
<220>
   <223> modified wildtye sequence
<400> 20
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Met Asn Leu Ser Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Val
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Val Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 21
   <211> 822
   <212> PRT
   <213> artificial
<220>
   <223> modified wildtype sequence
<400> 21
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Arg Asn Leu Val Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Val
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 22
   <211> 822
   <212> PRT
   <213> artificial
<220>
   <223> modified wildtype sequence
<400> 22
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Ala Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Glu His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Val
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 23
   <211> 822
   <212> PRT
   <213> artificial
<220>
   <223> modified wildtype sequence
<400> 23
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Ala Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asn Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Val
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 24
   <211> 822
   <212> PRT
   <213> artificial
<220>
   <223> modified wildtype sequence
<400> 24
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Ala Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asp Leu Leu Ser
   305                 310                 315                 320
   Thr Tyr Ser Val Ser Ser Asn Asp Glu Lys Leu Asp Arg Met Val Asn
                   325                 330                 335
   Ile Trp Asn Gln Tyr Gln Cys Met Val Thr Phe Asn Met Ser Arg Ser
               340                 345                 350
   Ala Ser Phe Phe Glu Thr Gly Ile Gly Arg Gly Met Gly Phe Arg Asp
           355                 360                 365
   Ser Asn Gln Asp Leu Leu Gly Phe Val His Leu Ile Pro Glu Arg Ala
       370                 375                 380
   Arg Glu Arg Ile Ile Asp Ile Ala Ser Thr Gln Phe Ala Asp Gly Ser
   385                 390                 395                 400
   Ala Tyr His Gln Tyr Gln Pro Leu Thr Lys Arg Gly Asn Asn Asp Ile
                   405                 410                 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Thr His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Val
               500                 505                 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Asn Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Met Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
       770                 775                 780
   Asn Ser Gly Val Asp Gly Ser Arg Gly Arg Leu Val Val Asp Gly Thr
   785                 790                 795                 800
   Pro Val Glu Gly Asn Leu Val Pro Tyr Ala Pro Ala Gly Ser Thr Val
                   805                 810                 815
   Arg Val Asp Val Thr Leu
               820
<210> 25
   <211> 822
   <212> PRT
   <213> artificial
<220>
   <223> modified wildtype sequence
<400> 25
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145 150 155 160
   Met Thr Asn Tyr Gln Arg Asn Leu Val Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Ile Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 26
   <211> 822
   <212> PRT
   <213> artificial
<220>
   <223> modified wildtype sequence
<400> 26
   Met Arg Tyr Gly His Phe Asp Asp Glu Ala Arg Glu Tyr Val Ile Thr
   1               5                   10                  15
   Thr Pro His Thr Pro Tyr Pro Trp Ile Asn Tyr Leu Gly Ser Glu Gln
               20                  25                  30
   Phe Phe Ser Leu Leu Ser His Gln Ala Gly Gly Tyr Ser Phe Tyr Arg
           35                  40                  45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Arg Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asp Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Ile Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
           675                 680                 685
   Lys Arg Ile Thr Pro Ala Tyr Arg Glu Asp Ile Ser Asp Val His Arg
       690                 695                 700
   Thr Glu Pro Tyr Val Tyr Ala Gln Met Ile Ala Gly Lys Glu Ala Val
   705                 710                 715                 720
   Arg His Gly Glu Ala Lys Asn Ser Trp Leu Thr Gly Thr Ala Ala Trp
                   725                 730                 735
   Asn Phe Val Thr Val Ser Gln Tyr Leu Leu Gly Val Arg Pro Glu Tyr
               740                 745                 750
   Asp Gly Leu Val Val Asp Pro Gln Ile Gly Pro Asp Val Pro Ser Phe
           755                 760                 765
   Thr Val Thr Arg Val Ala Arg Gly Ala Thr Tyr Glu Ile Thr Val Thr
       770                 775                 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 27
   <211> 822
   <212> PRT
   <213> artificial
<220>
   <223> modified wildtype sequence
<400> 27
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
   50 55 60
   Ala Asp Ala Gly Gly Arg Tyr Leu Tyr Val Asn Asp Gly Gly Asp Val
   65 70 75 80
   Trp Thr Pro Ser Trp Leu Pro Val Lys Ala Asp Leu Asp His Phe Glu
   85 90 95
   Ala Arg His Gly Leu Gly Tyr Ser Thr Ile Thr Gly Glu Arg Asn Gly
   100 105 110
   Val Arg Val Glu Thr Leu Phe Phe Val Pro Val Gly Glu Asn Ala Glu
   115 120 125
   Val Gln Lys Val Thr Val Thr Asn Thr Ser Asp Ser Tyr Lys Ser Leu
   130 135 140
   Thr Leu Phe Ser Phe Val Glu Phe Cys Leu Trp Asn Ala Gln Asp Asp
   145 150 155 160
   Met Thr Asn Tyr Gln Arg Asn Leu Val Ile Gly Glu Val Glu Val Glu
   165 170 175
   Gln Glu Ser Pro His Gly Ser Ala Ile Tyr His Lys Thr Glu Tyr Arg
   180 185 190
   Glu Arg Arg Asn His Tyr Ala Val Phe Ala Val Asn Thr Gln Ala Glu
   195 200 205
   Gly Phe Asp Thr Asp Arg Asp Thr Phe Val Gly Leu Tyr Asn Ser Leu
   210 215 220
   Gly Glu Ala Ala Val Pro Leu Lys Gly Glu Ser Ala Asn Ser Val Ala
   225 230 235 240
   Ser Gly Trp Tyr Pro Ile Gly Ser His Ser Val Ala Val Ser Leu Ala
   245 250 255
   Pro Gly Glu Ser Arg Glu Leu Val Tyr Val Leu Gly Tyr Val Glu Asn
   260 265 270
   Pro Asp Glu Glu Lys Trp Ala Asp Asp Ala Ala Gln Val Val Asn Lys
   275 280 285
   Glu Arg Ala His Ala Leu Leu Ser Arg Phe Ala Thr Ser Glu Gln Thr
   290 295 300
   Asp Ala Ala Phe Ala Ala Leu Lys Asp Tyr Trp Thr Asn Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
               420                 425                 430
   Ala Tyr Ile Lys Glu Thr Gly Asp Phe Ser Ile Leu Asp Glu Pro Val
           435                 440                 445
   Pro Phe Asp Asn Glu Pro Gly Ser Glu Val Pro Leu Phe Glu His Leu
       450                 455                 460
   Thr Arg Ser Phe Glu Phe Thr Val Glu His Arg Gly Pro His Gly Leu
   465                 470                 475                 480
   Pro Leu Ile Gly Arg Ala Asp Trp Asn Asp Cys Leu Asn Leu Asn Cys
                   485                 490                 495
   Phe Ser Thr Thr Pro Gly Glu Ser Phe Gln Thr Thr Glu Asn Gln Val
               500                 505                 510
   Gly Gly Val Ala Glu Ser Thr Phe Ile Ala Ala Gln Phe Val Leu Tyr
           515                 520                 525
   Gly Glu Gln Tyr Ala Glu Leu Ala Ala Arg Arg Gly Leu Ala Asp Val
       530                 535                 540
   Ala Asp Arg Ala Arg Gly His Val Ala Glu Met Arg Asp Ala Leu Leu
   545                 550                 555                 560
   Thr Asp Gly Trp Asp Gly Ser Trp Phe Leu Arg Ala Tyr Asp Tyr Tyr
                   565                 570                 575
   Gly Asn Pro Ile Gly Thr Asp Ala His Asp Glu Gly Lys Ile Trp Ile
               580                 585                 590
   Glu Pro Gln Gly Phe Ala Val Met Ala Gly Val Gly Val Gly Glu Gly
           595                 600                 605
   Pro Gln Asp Thr Asp Ala Pro Ala Ile Lys Ala Leu Asp Ser Val Asn
       610                 615                 620
   Glu Met Leu Ala Thr Asp His Gly Met Val Leu Gln Tyr Pro Ala Tyr
   625                 630                 635                 640
   Thr Thr Tyr Gln Val His Met Gly Glu Val Ser Thr Tyr Pro Pro Gly
                   645                 650                 655
   Tyr Lys Glu Asn Gly Gly Ile Phe Cys His Asn Asn Pro Trp Val Ile
               660                 665                 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
   805 810 815
   Arg Val Asp Val Thr Leu
   820
<210> 28
   <211> 822
   <212> PRT
   <213> artificial
<220>
   <223> modified wildtype sequence
<400> 28
   Met Arg Tyr Gly His Phe Asp Asp Glu Ala Arg Glu Tyr Val Ile Thr
   1 5 10 15
   Thr Pro His Thr Pro Tyr Pro Trp Ile Asn Tyr Leu Gly Ser Glu Gln
   20 25 30
   Phe Phe Ser Leu Leu Ser His Gln Ala Gly Gly Tyr Ser Phe Tyr Arg
   35 40 45
   Asp Ala Lys Met Arg Arg Leu Thr Arg Tyr Arg Tyr Asn Asn Ile Pro
       50                  55                  60
   Ala Asp Ala Gly Gly Arg Tyr Leu Tyr Val Asn Asp Gly Gly Asp Val
   65                  70                  75                  80
   Trp Thr Pro Ser Trp Leu Pro Val Lys Ala Asp Leu Asp His Phe Glu
                   85                  90                  95
   Ala Arg His Gly Leu Gly Tyr Ser Thr Ile Thr Gly Glu Arg Asn Gly
               100                 105                 110
   Val Arg Val Glu Thr Leu Phe Phe Val Pro Val Gly Glu Asn Ala Glu
           115                 120                 125
   Val Gln Lys Val Thr Val Thr Asn Thr Ser Asp Ser Tyr Lys Ser Leu
       130                 135                 140
   Thr Leu Phe Ser Phe Val Glu Phe Cys Leu Trp Asn Ala Gln Asp Asp
   145                 150                 155                 160
   Met Thr Asn Tyr Gln Arg Asn Leu Val Ile Gly Glu Val Glu Val Glu
                   165                 170                 175
   Gln Glu Ser Pro His Gly Ser Ala Ile Tyr His Lys Thr Glu Tyr Arg
               180                 185                 190
   Glu Arg Arg Asn His Tyr Ala Val Phe Ala Val Asn Thr Gln Ala Glu
           195                 200                 205
   Gly Phe Asp Thr Asp Arg Asp Thr Phe Val Gly Leu Tyr Asn Ser Leu
       210                 215                 220
   Gly Glu Ala Ala Val Pro Leu Lys Gly Glu Ser Ala Asn Ser Val Ala
   225                 230                 235                 240
   Ser Gly Trp Tyr Pro Ile Gly Ser His Ser Val Ala Val Ser Leu Ala
                   245                 250                 255
   Pro Gly Glu Ser Arg Glu Leu Val Tyr Val Leu Gly Tyr Val Glu Asn
               260                 265                 270
   Pro Asp Glu Glu Lys Trp Ala Asp Asp Ala Ala Gln Val Val Asn Lys
           275                 280                 285
   Glu Arg Ala His Ala Leu Leu Ser Arg Phe Ala Thr Ser Glu Gln Thr
       290                 295                 300
   Asp Ala Ala Phe Ala Ala Leu Lys Asp Tyr Trp Thr Asn Leu Leu Ser
   305 310 315 320
   Thr Tyr Ser Val Ser Ser Asn Asp Glu Lys Leu Asp Arg Met Val Asn
   325 330 335
   Ile Trp Asn Gln Tyr Gln Cys Met Val Thr Phe Asn Met Ser Arg Ser
   340 345 350
   Ala Ser Phe Phe Glu Thr Gly Ile Gly Arg Gly Met Gly Phe Arg Asp
   355 360 365
   Ser Asn Gln Asp Leu Leu Gly Phe Val His Leu Ile Pro Glu Arg Ala
   370 375 380
   Arg Glu Arg Ile Ile Asp Ile Ala Ser Thr Gln Phe Ala Asp Gly Ser
   385 390 395 400
   Ala Tyr His Gln Tyr Gln Pro Leu Thr Lys Arg Gly Asn Asn Asp Ile
   405 410 415
   Gly Ser Gly Phe Asn Asp Asp Pro Leu Trp Leu Ile Ala Gly Thr Ala
   420 425 430
   Ala Tyr Ile Lys Glu Thr Gly Asp Phe Ser Ile Leu Asp Glu Pro Val
   435 440 445
   Pro Phe Asp Asn Glu Pro Gly Ser Glu Val Pro Leu Phe Glu His Leu
   450 455 460
   Thr Arg Ser Phe Glu Phe Thr Val Thr His Arg Gly Pro His Gly Leu
   465 470 475 480
   Pro Leu Ile Gly Arg Ala Asp Trp Asn Asp Cys Leu Asn Leu Asn Cys
   485 490 495
   Phe Ser Thr Thr Pro Gly Glu Ser Phe Gln Thr Thr Glu Asn Gln Ala
   500 505 510
   Gly Gly Val Ala Glu Ser Thr Phe Ile Ala Ala Gln Phe Val Leu Tyr
   515 520 525
   Gly Glu Gln Tyr Ala Glu Leu Ala Ala Arg Arg Gly Leu Ala Asp Val
   530 535 540
   Ala Asp Arg Ala Arg Gly His Val Ala Glu Met Arg Asp Ala Leu Leu
   545 550 555 560
   Thr Asp Gly Trp Asp Gly Ser Trp Phe Leu Arg Ala Tyr Asp Tyr Tyr
   565 570 575
   Gly Asn Pro Ile Gly Thr Asp Ala His Asp Glu Gly Lys Ile Trp Ile
   580 585 590
   Glu Pro Gln Gly Phe Ala Val Met Ala Gly Val Gly Val Gly Glu Gly
   595 600 605
   Pro Gln Asp Thr Asp Ala Pro Ala Ile Lys Ala Leu Asp Ser Val Asn
   610 615 620
   Glu Met Leu Ala Thr Asp His Gly Ile Val Leu Gln Tyr Pro Ala Tyr
   625 630 635 640
   Thr Thr Tyr Gln Val His Met Gly Glu Val Ser Thr Tyr Pro Pro Gly
   645 650 655
   Tyr Lys Glu Asn Gly Gly Ile Phe Cys His Asn Asn Pro Trp Val Ile
   660 665 670
   Ile Ala Glu Thr Val Val Gly Arg Gly Gly Arg Ala Phe Asp Tyr Tyr
   675 680 685
   Lys Arg Ile Thr Pro Ala Tyr Arg Glu Asp Ile Ser Asp Val His Arg
   690 695 700
   Thr Glu Pro Tyr Val Tyr Ala Gln Met Ile Ala Gly Lys Glu Ala Val
   705 710 715 720
   Arg His Gly Glu Ala Lys Asn Ser Trp Leu Thr Gly Thr Ala Ala Trp
   725 730 735
   Asn Phe Val Thr Val Ser Gln Tyr Leu Leu Gly Val Arg Pro Glu Tyr
   740 745 750
   Asp Gly Leu Val Val Asp Pro Gln Ile Gly Pro Asp Val Pro Ser Phe
   755 760 765
   Thr Val Thr Arg Val Ala Arg Gly Ala Thr Tyr Glu Ile Thr Val Thr
   770 775 780
   Asn Ser Gly Val Asp Gly Ser Arg Gly Arg Leu Val Val Asp Gly Thr
   785 790 795 800
   Pro Val Glu Gly Asn Leu Val Pro Tyr Ala Pro Ala Gly Ser Thr Val
                   805                 810                 815
   Arg Val Asp Val Thr Leu
               820